# EUROPEAN PATENT SPECIFICATION

(11) **EP 3 440 578 B1**
(45) Date of publication and mention of the grant of the patent: **20.03.2024**
(21) Application number: 17718283.9
(22) Date of filing: 05.04.2017
(51) Int. Cl.: A61M 5/142, G16H 20/17

(54) **CONTROL OF A DRUG INFUSION DEVICE**
STEUERUNG EINER MEDIKAMENTENINFUSIONSVORRICHTUNG
COMMANDE D'UN DISPOSITIF DE PERFUSION DE MÉDICAMENT

(30) Priority: 08.04.2016 US 201662319849 P
(43) Date of publication of application: 13.02.2019
(73) Proprietor: ICU Medical, Inc., San Clemente, CA 92673 (US)
(72) Inventor: PACE, William Bruce, San Clemente, CA 92673 (US); RINDA, Jeffrey Eugene, San Clemente, CA 92673 (US); MARINO, Angela, San Clemente, CA 92673 (US); WILLEY, Suzanne, San Clemente, CA 92673 (US)
(74) Representative: Invent Horizon IP
(86) International application number: PCT/US2017/026216
(87) International publication number: WO 2017/176928

(56) References cited:
- US-A- 5 317 506
- US-A1- 2008 065 007
- US-A1- 2015 379 237

## Description

### CROSS-REFERENCE TO RELATED APPLICATIONS

This application claims the benefit of U.S. Provisional Application No. 62/319,849, filed April 8, 2016, entitled CONTROL OF A DRUG INFUSION DEVICE. The benefit of priority to the foregoing application is claimed under the appropriate legal basis, including, without limitation, under 35 U.S.C. §119(e).

### BACKGROUND OF THE INVENTION

Drug infusion devices may be used for controlled delivery of a variety of different types of drugs to a patient. Typically, a doctor orders a prescription for a particular drug with allowable dose amounts and a dosing schedule for the patient. Then the drug infusion device is programmed or configured with various parameters that are determined in accordance with the prescription, and a container of the drug is attached to the drug infusion device. The drug infusion device then delivers, or infuses, the drug from the container to the patient according to the programmed parameters.

The parameters typically call for a continuous delivery mode for the drug and/or a repeatable as-needed patient-controlled delivery mode. The continuous delivery mode is automatic, meaning there is no need for the patient, nurse or other person to intervene during normal infusion operations after the programming of the drug infusion device until the container runs empty. The patient-controlled delivery mode is generally controlled, for example, by manually activating the release of a single-dose at a time to the patient.

Certain considerations are generally taken into account for the design of the drug infusion devices and the control features thereof. One such consideration is that it should be possible to program the drug infusion device and begin delivering the drug relatively quickly. For example, a patient who is suffering acute distress may need to begin experiencing some relief as quickly as possible, so the time needed for setting up, programming or configuring the drug infusion device must be minimized. Another consideration is that it should be fairly easy or straightforward to program or otherwise operate the drug infusion device. Otherwise, a user may be more likely to make mistakes when setting-up/programming the drug infusion device or take an unacceptably long time to get the drug infusion started. A further consideration is that the safety of the patient should be of paramount importance. Therefore, accuracy of the setting-up, programming or configuring of the drug infusion device should not be sacrificed for a quick or easy operation thereof, since any mistake could potentially be devastating for the health or well-being of the patient. Other considerations may include a high accuracy for drug delivery rate/amount and a low mean-time-between-failure for the overall device, among other potential issues.

US 2015/379237 discloses systems and methods for analyzing an auto-program after it has been received by the infusion device, so that error messages can be displayed to the user. The infusion device verifies the infusion program settings against the installed drug library.

US 2008/065007 discloses a menu driven reprogrammable drug pump provided with a communications port to allow a generic pump to be programmed with a desired pump application. Automated testing of the pump is by a closed loop testing system.

US 5317506 discloses an infusion management and pumping system. The infusion pumping system is responsive to remote or biofeedback instructions to alter the planned infusion program.

### SUMMARY

The invention is defined by the independent claims. Preferable embodiments are defined in the dependent claims.

### BRIEF DESCRIPTION OF THE DRAWINGS

FIG. 1 depicts an embodiment of a system for administering a medication to a patient in accordance with an embodiment of the present invention.
FIG. 2 depicts an embodiment of a drug infusion device for use in a medication administering system.
FIG. 3 depicts an embodiment of an example workflow process for operating a drug infusion device.
FIG. 4 depicts an embodiment of an example workflow process for operating a drug infusion device.
FIG. 5 depicts an embodiment of an example workflow process for operating a drug infusion device.
FIG. 6 depicts an embodiment of an example workflow process for operating a drug infusion device.
FIG. 7 depicts an embodiment of an example workflow process for operating a drug infusion device.
FIG. 8 depicts an embodiment of an example workflow process for operating a drug infusion device.
FIG. 9 depicts an embodiment of an example workflow process for operating a drug infusion device.
FIG. 10 depicts an embodiment of an example workflow process for operating a drug infusion device.
FIG. 11 depicts an embodiment of an example workflow process for operating a drug infusion device.
FIG. 12 depicts an embodiment of an example workflow process for operating a drug infusion device.

### DETAILED DESCRIPTION

In order that this invention may be better understood, the following examples are set forth. These examples are for purposes of illustration only and are not to be construed as limiting the scope of the invention in any manner.

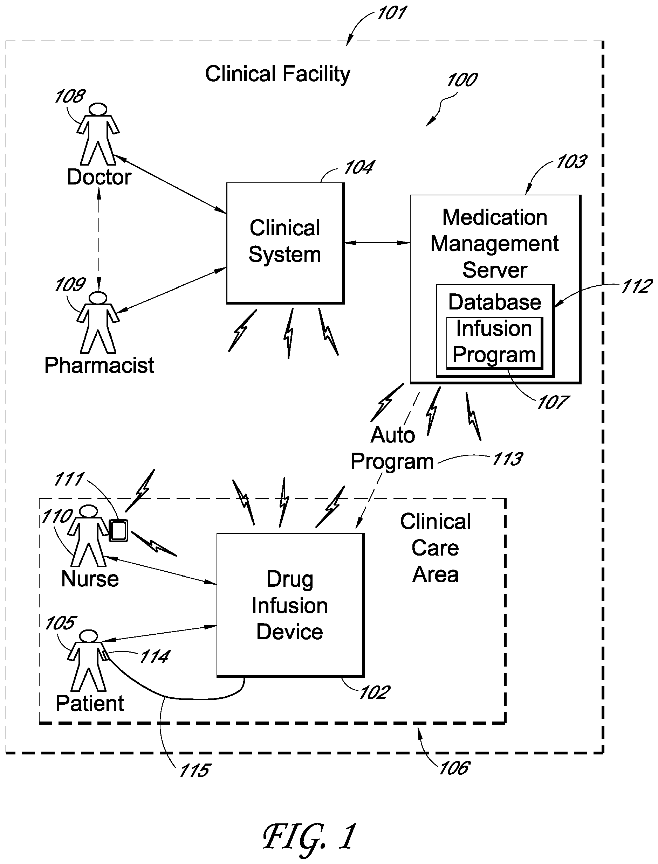

An example medication administering system 100, such as may be used within a clinical facility 101, is shown in FIG. 1 in accordance with an embodiment of the present invention. The medication administering system 100 generally includes a drug infusion device 102 in communication with a medication management server 103 and a system of computers, workstations, servers, and network communication devices (the clinical system) 104 within the clinical facility 101. Additionally, the medication administering system 100 may further include other components or combinations of components not shown for simplicity of explanation.

In accordance with various embodiments, the drug infusion device 102 is capable of being connected to a patient 105 within a clinical care area 106 of the clinical facility 101 and delivering, infusing or providing a drug or medication to the patient 105 in accordance with an infusion program 107 after configuration of the drug infusion device 102 according to parameters specified by the infusion program 107. In one embodiment, the clinician can manually enter parameters for the infusion program 107 into the device 102 through a user interface and/or keypad described below. In some other embodiments, the infusion program 107 can be electronically delivered to the drug infusion device 102 by the medication management server 103 and/or the clinical system 104 via an auto program message (auto program) 113. The auto program feature is advantageous when it is stored in the drug infusion device 102, because it can be used by the drug infusion device 102 to automatically set infusion parameters or configure itself without interference or intervention by a user or clinician, except primarily for performing appropriate safety checks. Additionally, in some embodiments, the drug infusion device 102 includes various operation and control features that render the drug infusion device 102 capable of being programmed, set up or configured relatively quickly, so that the drug infusion device 102 can begin delivering the drug with very little lead time or delay. Also, the drug infusion device 102 includes additional operation and control features that enable redundant safety checks to minimize, mitigate or eliminate the potential for some types of mistakes occurring in the setting-up, programming or configuring of the drug infusion device 102. These features also enhance the ease with which the drug infusion device 102 can be programmed, further mitigating the potential for mistakes or human error. These and other features will be described in more detail below.

The clinical facility 101 generally represents any appropriate facility or environment in which medical care may be provided, such as a hospital, a nursing home, a hospice, an emergency clinic, a doctor's office, a field or mobile clinic, a recovery center, etc. In some embodiments, the clinical facility 101 may even represent a home for a patient 105 receiving medical care at home. Any appropriate number and types of medical care providers and management and staff personnel (e.g., doctors 108, pharmacists 109, nurses 110, clinicians, technicians, device operators, etc.) may perform medical care or support duties within or in association with the clinical facility 101, including operating the drug infusion device 102 to deliver a drug to the patient 105 (e.g., through a catheter 114 and a drug administration set 115 attached to the patient 105 and the drug infusion device 102). Additionally, the clinical facility 101 generally includes one or more of the clinical care areas 106.

The clinical care area 106 generally represents any appropriate designated area in which medical care may be provided within the clinical facility 101. For example, the clinical care area 106 may be an intensive care unit or ward, a common patient room, an operating room, a recovery room, an examining room, a medical testing room, a long term care ward, a patient's home, etc. Different clinical care areas 106 may be associated with different drugs or drug dosages that are allowed to be provided to the patient 105 when the patient 105 is receiving medical care within each of the different types of clinical care areas 106. Thus, the infusion program 107 may be limited to certain parameters depending on the clinical care area 106 in which it is being used.

The clinical system 104 generally represents a computerized system of any appropriate number, type and combination of computers, workstations, terminals, servers, handheld devices, computers on wheels, and wired and wireless network communication devices within or associated with the clinical facility 101. In some embodiments, for example the clinical system 104 represents components of a Hospital Information System (HIS) or Electronic Medical Records (EMR) Management System. Various medical care providers and management and staff personnel may use various terminals of the clinical system 104 to manage some of the medical care functions for the patient 105 related to the drug infusion device 102, as described below, as well as many other medical and/or business functions of the clinical facility 101. For example, in some embodiments, the doctor 108 may use a desktop, notebook, tablet, handheld computer or other terminal of the clinical system 104 to enter into the clinical system 104 a prescription for a desired drug to be provided to the patient 105. Alternatively, the doctor 108 may simply write the prescription down, and the nurse 110, pharmacist 109 or other clinician having appropriate authority may enter the prescription into the clinical system 104. The prescription may then be routed through the clinical system 104 to a terminal for an appropriate pharmacist 109 or other clinician who generates a formal order for the drug to be provided to the patient 105, based on the prescription. The formal order is processed through the clinical system 104 and the medication management server 103 to generate the infusion program 107 for eventual electronic transmission to, and programming of, the drug infusion device 102, as described below.

In other embodiments, the drug infusion device 102 may be programmed manually, e.g., by the nurse 110 or other clinician, in accordance with the prescription or order produced by the doctor 108. In this case, the infusion program 107 may be generated at the drug infusion device 102, as described below, instead of at the clinical system 104 and the medication management server 103. To do so, the drug infusion device 102 may be directly operated with built-in control features (described below). Alternatively, the drug infusion device 102 may be operated remotely, e.g., with a remote control or handheld device 111 that has a wireless connection directly to the drug infusion device 102 or indirectly through the wireless capabilities of the clinical system 104 to the drug infusion device 102. In some embodiments, however, these manual programming capabilities can be bypassed by the above mentioned technique for generating the infusion program 107 through the clinical system 104 and the medication management server 103 and electronically transmitting the infusion program 107 to the drug infusion device 102.

The medication management server 103 generally represents one or more computer servers and workstations or terminals for accessing the computer servers, either onsite at the clinical facility 101 or offsite at a remote computing center (or a combination of both onsite and offsite computing facilities). Additionally, the medication management server 103 maintains a database 112 or drug library of data used for managing the proper functioning of the drug infusion device 102, among other data. For example, the database 112 may store drug data describing the various drugs that the drug infusion device 102 may deliver to the patient 105 and allowable parameters for programming the drug infusion device 102 under various conditions or within the various clinical care areas 106. In response to orders received from the clinical system 104, the medication management server 103 may also generate the infusion programs 107 for the patients 105 and store the infusion programs 107 in the database 112. When called upon to program the drug infusion device 102 for the patient 105, the medication management server 103 transmits the desired infusion program 107 through the clinical system 104, e.g., through a wired and wireless network communication subsystem, to the drug infusion device 102.

The medication management server 103 and the clinical system 104 are connected together and their functions coordinated such that the formal order generated by the clinician through the clinical system 104, as mentioned above, is in a format with which the parameters for programming the drug infusion device 102 can be determined. With specific allowable parameters provided by the medication management server 103 and the order generated through the clinical system 104, therefore, the infusion program 107 for programming or configuring the drug infusion device 102 is generated in accordance with the original prescription provided by the doctor 108. The infusion program 107 specifies the exact parameters by which the drug may be delivered by the drug infusion device 102 to the patient 105. The infusion program 107 is electronically transmitted to the drug infusion device 102, and the drug infusion device 102 is configured accordingly, as described below.

In addition, where appropriate, and where it supplements without contradicting the disclosure herein, some of the components, features, and/or functions of the medication administering system 100 may be described with respect to similar components and features shown and described in US Patent No. 8768719 82, which is assigned to the same assignee as that of the present invention.

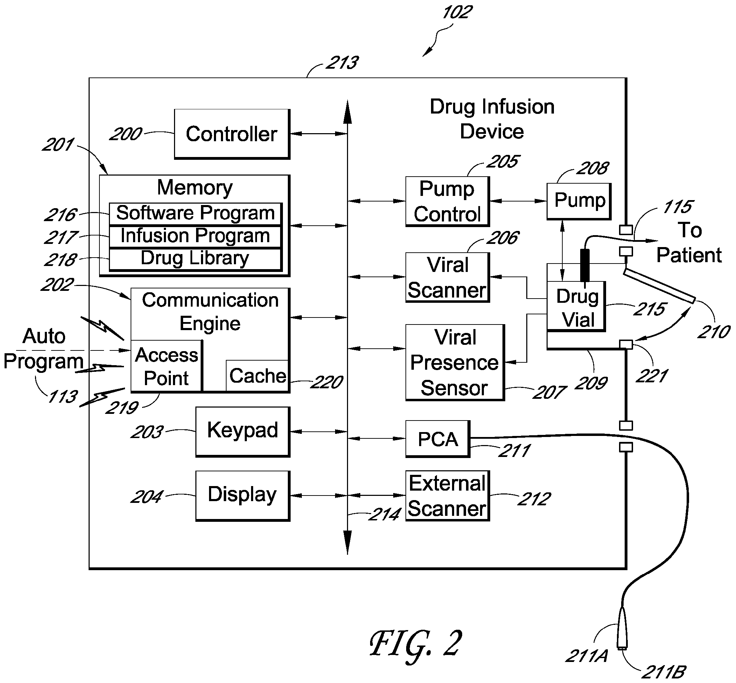

In some embodiments, as shown in a simplified or idealized illustration in FIG. 2, the drug infusion device 102 generally includes a controller 200, a memory 201, a communication engine 202, a keypad 203, a display 204, a pump control logic 205, a vial scanner 206, a vial presence sensor 207, a pump unit 208, a lockable, securable, or sealable receptacle space 209, a door 210, a PCA (patient controlled analgesia) control unit 211, and an optional external scanner 212, all generally contained within, if not exposed on the outside of, a housing 213. Additionally, some or all of these components are electrically connected together by an internal communication system 214. Furthermore, a container of the drug, such as a drug vial 215, may be received into the receptacle space 209 within the housing 213. A patient actuatable pendant 211 A is connected to the PCA control unit 211 and extends from the housing 213 so that the patient can request delivery of the medication themselves, e.g., by pressing a button 211 B on the pendant 211A.

In some embodiments, some of these components 200-215, or the features or functions described, may be combined into, or distributed among, a different number or combination of actual components. Additionally, in some embodiments, the drug infusion device 102 may further include other components, features or functions or combinations thereof (e.g., a battery, a power supply, a speaker, a pole clamp, etc.) not shown for simplicity of explanation. Therefore, the specific components 200-215 shown and described are simplified or idealized components that are provided for illustrative and explanatory purposes only and are not meant to limit the scope of the invention, unless otherwise specified.

The controller 200 generally represents one or more appropriate programmable microprocessors, microcontrollers or central processing units (CPU). The controller 200 controls various components, features or functions of the drug infusion device 102 in accordance with programmed instructions and data stored in and received from the memory 201. The programmed instructions and data may be replaced, updated or enhanced by electronically transmitting new or revised programmed instructions and data from the medication management server 103, e.g., through the network communications functions of the clinical system 104. For example, the controller 200 may control the various components, features and functions associated with configuring the drug infusion device 102 with the infusion program 107, whether manually entered by a clinician or automatically received from the medication management server 103, as described below. Additionally, the controller 200 may handle tasks associated with monitoring the delivery of the drug to the patient 105, logging occurrences of events/alarms during delivery of the drug or changes in the status of the drug infusion device 102, and reporting such event, alarm, and status information to the medication management server 103.

The internal communication system 214 generally represents one or more appropriate communication components through which the various other components of the drug infusion device 102 may transmit and receive commands, data or signals between each other. In some embodiments, not all of the components of the drug infusion device 102 use the same communication components or protocols for such communications. Thus, some combinations of the components of the drug infusion device 102 may have communication capabilities that are separate from other components of the drug infusion device 102.

The memory 201 generally represents one or more appropriate computer or electronic memory devices, such as SRAM or DRAM memory modules, flash memory devices, mass storage devices (e.g., magnetic, optical, etc.), registers, field programmable devices, etc. The drug infusion device 102 may use a variety of the different types of electronic memory devices, e.g., each for a different specific purpose. In general, though, the memory 201 stores the programmed instructions and data used by the controller 200 and files that have been received from or are to be transmitted to the medication management server 103 or the clinical system 104. For example, in some embodiments, the memory 201 stores a software program 216, an infusion program 217, and a drug library 218, among other programmed instructions and data not shown for simplicity, for use in cooperation with the controller 200.

The software program 216, defining the workflow aspects of the drug infusion device 102, generally represents the programmed instructions and data used by the controller 200 during a workflow to initialize the drug infusion device 102 for operation, configure the drug infusion device 102 for delivering the drug to the patient 105, and monitor the drug delivery process, among other potential functions not shown for simplicity. The infusion program 217 generally represents one or more program drug identifiers, such as the name of the drug, the concentration of the drug, and the parameters used to configure the drug infusion device 102 during the execution of the workflow for delivering the drug to the patient 105. Under the auto program feature, the auto program 113 is received through the communication engine 202 and stored as the infusion program 217. The auto program 113 generally represents a message or file containing the program drug identifiers and other parameters used to automatically configure the drug infusion device 102 for delivering the drug to the patient 105 in accordance with the infusion program 107 upon electronic transmission of the infusion program 107 (via the message of the auto program 113), thereby bypassing some manual programming steps typically performed by the nurse 110 or clinician. Thus, in some embodiments, the infusion program 217 refers generically to the parameters used for drug delivery configuration; whereas the auto program 113 refers more specifically to the message or file used in a situation where the automatic configuration capability is enabled and performed. In other words, the infusion program 107, or portions thereof, is the payload carried by the electronically transmitted message or file of the auto program 113 from the medication management server 103 to the drug infusion device 102 to form the infusion program 217.

The communication engine 202 generally represents one or more appropriate components used to transmit and receive network packets to and from the medication management server 103 or the clinical system 104. Thus, the communication engine 202 includes a network access point 219 for establishing a network connection with the network communication devices of the medication management server 103 or the clinical system 104. In some embodiments, particularly for mobile designs of the drug infusion device 102, the network connection is wireless, such as WiFi, Bluetooth, etc. However, wired network connections may also be used, such as Ethernet, IEEE 1395 FireWire, etc. Additionally, the communication engine 202 may also include a cache memory 220, typically for the temporary storage of network packets and the messages converted from, or to be converted to, a protocol for transmitting and receiving the network packets. For example, when the drug infusion device 102 receives the auto program 113 from the medication management server 103 or the clinical system 104, these components 219 and 220 (and other components, such as a processor circuitry) of the communication engine 202 handle the receipt and packet conversion and then send it through the internal communication system 214 for storage in the memory 201 as the infusion program 217. In a reverse process, log files and other messages to the medication management server 103 and/or the clinical system 104 are transmitted out.

The keypad 203 and the display 204 represent built-in control features or interfaces for operating the drug infusion device 102. For example, the display 204 generally represents one or more display screen devices (e.g., a flat panel display, an LCD, an LED, etc.) that may be used to present information and menu options (e.g., status, instructions, requests, alerts, alarms, etc.) to the user (e.g., nurse 110 or other clinician) during setup, configuration and/or operation of the drug infusion device 102. Additionally, the keypad 203 generally represents one or more appropriate input devices involving push buttons, such as an alphanumeric keyboard, an enter button, a set of selection keys, etc. With the keypad 203, the user can input instructions or make selections for configuring or operating the drug infusion device 102, e.g., in response to the information or menu options presented on the display 204, as described below. Some of the push buttons may be aligned with areas of the display 204, so that the user can select menu options presented within these areas. In some embodiments, the display 204 may be touchpad, so it can also double as an input device.

The receptacle space 209 represents a region within the housing 213 that is accessible through the door 210 and in which the drug vial 215 may be inserted or loaded, so that the drug in the drug vial 215 may be drawn out and delivered to the patient 105. Additionally, since it is desirable from a safety and security standpoint for only a properly authorized clinician to operate the drug infusion device 102, the door 210 has a lock to prevent an unauthorized person from attempting to remove the drug vial 215. Thus, the user may open the door 210, insert the drug vial 215 into the receptacle space 209 (e.g., into a cradle), and close and lock the door 210. Furthermore, a door sensor 221 is provided in order to detect whether the door 210 is properly closed, latched and/or locked, because it is desirable from a safety and security standpoint to enable some of the operational functions of the drug infusion device 102 only when the door 210 is properly closed and/or locked and the drug vial 215 cannot be removed. Thus, a signal indicating whether the door 210 is properly closed and/or locked may be sent from the door sensor 221 to the controller 200, which may then affect the workflow, as described below.

The vial presence sensor 207 represents any appropriate component that can detect whether the drug vial 215 is properly inserted, or loaded, into the receptacle space 209. In some embodiments, for example, the vial presence sensor 207 may be a set/reset trigger button disposed within the receptacle space 209 in such a manner that when the drug vial 215 is properly inserted, the trigger button is depressed. Upon depressing the trigger button, a vial detect signal is activated, which indicates that the drug vial 215 is in its proper insertion position for use with the drug infusion device 102. Notification of the signal activation is provided to the controller 200 to affect the workflow, as described below. Also, when the drug vial 215 is removed, or just slightly pulled away from its proper insertion position, the trigger button is released, thereby deactivating the vial detect signal. Notification of the signal deactivation, even if occurring only momentarily, is provided to the controller 200 to affect the workflow, as described below.

In some embodiments, the vial presence sensor 207 and the vial detect signal may be used to trigger a reset of the workflow of the controller 200 to an appropriate point at which the configuration of the drug infusion device 102 can be restarted. In this case, the notification of the deactivation of the vial detect signal may be considered to be a "vial-reset signal" that triggers a "vial-reset" condition or function. In some embodiments, the point within the workflow to which the workflow is reset is after an initial power-on self-test and optionally bypasses any other early steps of the workflow that may be unnecessary in a reset event, as described below. The vial-reset function thus enables the clinician, whenever it may become necessary, to be able to restart the configuration of the drug infusion device 102 by simply removing and reinserting the drug vial 215 and without having to completely restart the entire drug infusion device 102, as would occur if the clinician cycled the power off and on for the drug infusion device 102. The vial-reset function, therefore, may save valuable time when the clinician determines that an error has been made during the configuration process, and the best solution is simply to start over, but the patient 105 needs the drug delivery as soon as possible.

In some embodiments, the reset function may be enabled by some means other than using the vial detect signal. For example, a separate reset button may be provided on the keypad 203. However, for safety and security purposes, it is not desirable to have such a reset button be easily accessible. Otherwise, an unauthorized person might accidentally or maliciously reset the drug infusion device 102, thereby causing severe problems for the patient 105. By using the vial presence sensor 207 as a reset button, i.e., hiding the reset button inside the receptacle space 209 behind both the lockable door 210 and the drug vial 215, it is possible to ensure that only a properly authorized clinician will trigger a reset.

The vial scanner 206 generally represents any appropriate component that can read or scan information from the drug vial 215. For example, the drug vial 215 may be provided with an external barcode or an RF 10 tag that encodes "one or more vial drug identifiers" that identify the name of the drug contained in the drug vial 215 and one or more parameters, such as the amount and/or concentration of the drug. The vial scanner 206 is disposed in the receptacle space 209, so the vial scanner 206 reads the vial drug identifiers when the drug vial 215 is inserted into its proper insertion position in the receptacle space 209. In some embodiments, the reading or scanning is triggered by the vial detect signal generated by the vial presence sensor 207 upon insertion of the drug vial 215. The vial drug identifiers are provided to the controller 200 for use in the workflow. The read or scanned vial drug identifiers are used by the controller 200 to select drug-related operational limits from the drug library 218, which has also been transmitted to the memory 201 by the medication management server 103 or clinical system 104.

It may be assumed that the clinician operating the drug infusion device 102 will check the drug vial 215 against the order or doctor's prescription before inserting the drug vial 215 into the receptacle space 209. However, it is still possible, for example, for the clinician to set the drug vial 215 down and subsequently pick up a wrong drug vial 215 or otherwise make some kind of mistake that results in the wrong drug vial 215 being inserted into the receptacle space 209. With the vial scanning feature enabled by the vial scanner 206 disposed within the receptacle space 209 and triggered by the vial detect signal, on the other hand, it can be considered fairly certain that the drug identified by the scanned vial drug parameters/identifiers has in fact been loaded into the drug infusion device 102. In this manner, a redundant safety and security check is provided by this vial scanning feature to ensure that the correct drug is being delivered to the patient 105 at the correct concentration and dosage.

The pump unit 208 generally represents any appropriate combination of one or more components that can cause the drug to be drawn out of the drug vial 215 and delivered to the patient 105, e.g., through the catheter 114 and the drug administration set 115, e.g., a series of tubes, connectors and needles. For example, the pump unit 208 may represent an actuator and piston, wherein the actuator plunges the piston into the drug vial 215 to generate a pressure differential that causes the drug to flow through the drug administration set 115. Thus, when the drug vial 215 has been properly inserted into the receptacle space 209 and the door 210 has been closed and locked, the pump unit 208 has access to the drug in the drug vial 215, so when the drug infusion device 102 has been programmed, the pump unit 208 can draw out the drug and deliver it to the patient 105 through the catheter 114 and the drug administration set 115.

The pump control logic 205 generally represents any appropriate components that can directly control the pump unit 208 in accordance with the infusion parameters of the infusion program 217, such as a motor control unit, registers, state machines, motor driver, etc. In some embodiments, the infusion parameters are entered into the pump control logic 205. With these parameters, the pump control logic 205 is configured to control the pump unit 208 to deliver the drug at the desired flow rate, volume, time interval, etc. In some embodiments, the pump control logic 205 may be the same micro control unit that comprises the controller 200.

The PCA control unit 211 generally represents any appropriate components that enable the patient 105 to select when to receive a single PCA dose of the drug, such as an as-needed dose of pain medication. In some embodiments, the PCA control unit 211 includes the PCA patient pendant cord 211A with the button 211 B that the patient 105 may press to receive the PCA dose. The PCA control unit 211, thus, provides a signal to the controller 200 or the pump control logic 205 to activate the PCA dose. The parameters of the infusion program 217 describe the amount of the PCA dose, a lockout time interval that the patient 105 must wait before being able 10 to repeat the PCA dose, and/or a time period during which the patient 105 may receive the PCA dose.

The external scanner 212 generally represents any appropriate one or more components for scanning a patient wristband for a patient ID and/or an identifier such as a barcode or RF ID tag on the drug vial 215 for a vial ID before inserting the drug vial 215 into the receptacle space 209. With this scan, both the patient 105 and the drug vial 215 can be matched with the infusion program 217. In this manner, the clinician has another redundant check with which to be sure that the correct patient is about to receive the correct medication. Additionally, the patient ID can be sent to the medication management server 103 and/or the clinical system 104, so that the medication management server 103 and/or the clinical system 104 has data indicating which drug infusion device 102 is associated with the patient 105. Once the medication management server 103 has the data associating the drug infusion device 102 with the patient 105, the medication management server 103 can electronically transmit the infusion program 107 for the patient 105 to the correct drug infusion device 102.

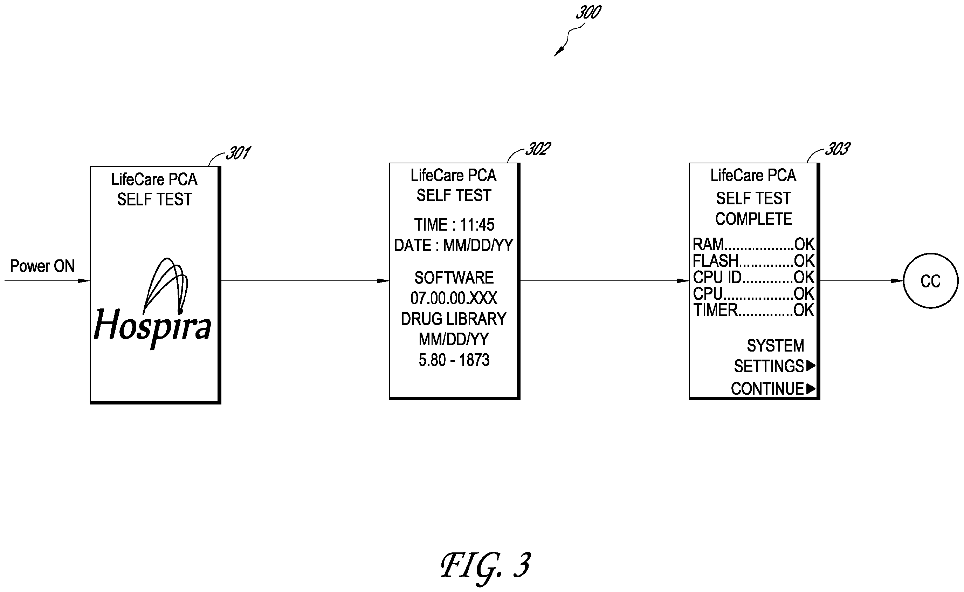

FIGS. 3-12 show portions of a simplified flowchart of an example process flow for the software program 216 for starting and configuring the drug infusion device 102 and further operating the drug infusion device 102 to deliver the drug to the patient 105, in accordance with an embodiment of the present invention. Various simplified example screenshots of display screens and menu screens are shown in the example process flow to illustrate status, feedback, instructions and/or prompts provided to the user or clinician at various stages or steps of the software program 216 workflow. The user may select various menu options using the keypad 203. These simplified flowchart portions are provided for illustrative and explanatory purposes only, since other specific display and menu screens, other specific process flow steps, and other specific combinations or orders of steps may be used in other embodiments to achieve generally the same results.

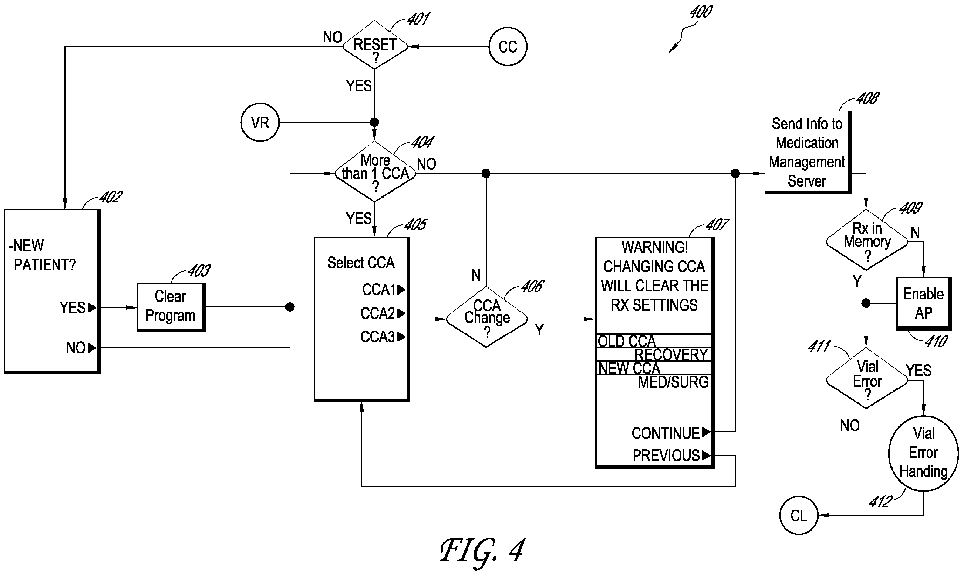

FIG. 3 shows an example start-up and power-on self-test portion 300 of the example process flow for the drug infusion device 102. Shortly after the drug infusion device 102 is turned on (power on), an example display screen 301 is presented on the display 204 to indicate that the drug infusion device 102 is operational and starting the self-test. Then an example display screen 302 presents certain general information about the drug infusion device 102 to the user, such as the time and date, a software version (e.g., for the software program 216), and a drug library version (e.g., the drug library 218, which is a local copy of at least a portion of the drug library in the database 112), among other possible types of information. Then, optionally, an example display screen 303 can present some results of the self-test, such as the pass/fail condition or status of certain components (e.g., RAM and flash components of the memory 201, the controller or CPU 200, and a timer). The example display screen 303 also prompts the user to select either "system settings" to set some types of general operating parameters of the drug infusion device 102 or "continue" to proceed with the next portion of the process flow (designated herein as "CC," FIG. 4) for configuring the drug infusion device 102 for an infusion program 217. If the screen 303 is omitted, the system settings and continue prompts or functionality can be relocated to another 25 screen, such as screen 302 for example.

FIG. 4 shows a portion 400 of the process flow (typically following portion 300) that is generally for designating a patient and a clinical care area (CCA). Each time the drug infusion device 102 is used, it may already have the infusion program 217 stored in the memory 201 from the previous usage. Sometimes the drug infusion device 102 may be expected to continue to use the previous infusion program 217. However, under some conditions, it may be considered prudent to simply start over and reset the drug infusion device 102. Such conditions may apply, for example, if the drug infusion device 102 has been turned off for an extended period time (perhaps four hours or more) or is being prepared for use on a new (different) patient, among other possible scenarios. Therefore, at a query 401, it is determined whether any of these conditions apply, such that the drug infusion device 102 should be completely reset from a previous use. If so, then the previous infusion program 217 is deleted or cleared at about this point.

If the determination at 401 is negative, then it may be possible to use the previous infusion program 217 (if present) if the patient 105 has not changed. Therefore, a menu screen 402 is presented to prompt the user to indicate whether the patient 105 is new. If so, then the infusion program 217 is cleared at 403.

Even if the patient 105 is the same as the previous one, it is generally hospital good practice to clear the previous infusion program 217 if the clinical care area 106 is different from the previous use. If there is more than one clinical care area 106, as determined at 404, then it is possible for it to change. If there is only one clinical care area 106, then it is selected automatically at 404, and an ID for the clinical care area 106 is stored in the memory 201. On the other hand, if there is more than one clinical care area 106, as determined at 404, a menu screen 405 is presented to prompt the user to indicate the clinical care area 106 in which the drug infusion device 102 is to be used. When the user selects (at 405) the clinical care area 106, the ID for the clinical care area 106 is stored in the memory 201. If the selected clinical care area 106 is different from the previous one, as determined at 406, then a menu screen 407 is presented to warn or remind the user that changing the clinical care area 106 will result in clearing the infusion program 217 ("Rx in memory" or program settings in FIG. 4). The names of or descriptors for the old and the new clinical care areas 106 are displayed for the user to review. Additionally, the user is prompted to select either to "continue" with the change to the clinical care area 106 or to return to the "previous" menu screen 405 to correct the selection of clinical care area 106 and avoid deleting the existing infusion program 217 if possible. On the other hand, if the selected clinical care area 106 has not changed, as determined at 406, then the menu screen 407 is skipped.

If the determination at 401 is positive (i.e., that the drug infusion device 102 should be automatically reset) or the patient 105 is new (as indicated at 402), then it is still necessary to select the clinical care area 106, as described previously. In some embodiments, there is no preexisting ID for the clinical care area 106 stored in the memory 201, if any such data was cleared along with deletion of the previous infusion program 217. In other embodiments, the ID for the clinical care area 106 is not cleared, but it is still necessary to either confirm or change it through 404-407. Additionally, if the vial-reset ("VR") condition has occurred, as mentioned above, then a point between 401 and 404 is one possible point within the process flow of the software program 216 to which the workflow may be reset, thereby avoiding the start-up and power-on self-test portion 300 and bypassing at least the receiving of the indication at 402 of whether the patient to be served by the drug infusion device is a new patient. (For the vial-reset condition, returning the process to this point results in restarting the program review aspects of the process, e.g., with subsequent options being generally to retain, clear or modify the existing infusion program 15 217.) In any of these cases, if there is only one clinical care area 106, as determined at 404, then it is selected automatically, and the ID for the clinical care area 106 is stored in the memory 201. On the other hand, if there is more than one clinical care area 106, as determined at 404, the menu screen 405 is presented to prompt the user to indicate the clinical care area 106 in which the drug infusion device 102 is located or to be used. When the user selects (at 405) the clinical care area 106, the ID for the clinical care area 106 is stored in the memory 201. However, since there was no ID for a previous clinical care area 106 stored in the memory 201 in any of these situations, the determination at 406 is necessarily negative, and so the menu screen 407 is skipped.

It is generally preferable in some embodiments for the medication management server 103 to be able to send the auto program 113 to the drug infusion device 102 as early as possible in the process flow, even if the process flow has not yet reached a point at which it can use the infusion program 107. In this manner, it can be assured that the drug infusion device 102 is not often left waiting for the auto program 113 to arrive, but can instead begin to benefit as soon as possible from the expedited process flow. On the other hand, it is generally not preferable in some embodiments for the drug infusion device 102 to receive the auto program 113 from the medication management server 103 before the clinical care area 106 has been identified. Otherwise, the drug infusion device 102 may have stored an auto program 113 that it is not valid for the clinical care area 106. In some embodiments according to the claimed invention, therefore, after the clinical care area 106 has been identified, the drug infusion device 102 is simply enabled with a capability to receive the auto program 113. This capability further depends on whether the preexisting infusion program 217 is still stored, or programmed into, the drug infusion device 102. Confusion between conflicting infusion programs can be avoided, therefore, if the drug infusion device 102 is disabled from the capability of storing the received auto program 113 as long as the previous infusion program 217 is still present in memory.

At the point after 401-407, the clinical care area 106 has been identified within the drug infusion device 102 and, in some of the above described situations, the infusion program 217 is not present. For the medication management server 103 to properly transmit the auto program 113, however, the drug infusion device 102 needs to be associated with the patient 105. Furthermore, a clinical care area 106 needs to be selected on the drug infusion device 102 and an identifier for the clinical care area 106 transmitted to the server 103 at 408 to identify the area the device 102 is located in or being prepared for use in. It is typical in some embodiments to have also identified the patient 105 within the clinical system 104 by this point, e.g., by scanning the wristband for the patient 10, as mentioned above. The patient 10, the drug 10 on the drug container, and an 10 for the drug infusion device 102 are collected by a scanner such as in the handheld device 111, vial scanner 206, or the external scanner 212, and then may be sent to the medication management server 103 along with an infusion program 107. With this 10 information properly associating the drug or drug container, the patient 105, and the drug infusion device 102 together, the medication management server 103 can determine whether it has an infusion program 107 for the identified patient 105 that can be used in the identified device 102. For accuracy of drug delivery reports, it also helps the medication management server 103 to know the clinical care area 106 of the drug infusion device. At any rate, the medication management server 103 can send the proper infusion program 107 via an auto program message 113 to the identified drug infusion device 102.

However, the infusion program 107 might not be sent immediately upon the medication management server 103 receiving the various ID information. The delay may be caused by a variety of reasons. For example, the infusion program 107 may simply not be ready yet, or high network traffic may slow network communications through the clinical system 104. Therefore, if the infusion program 217 (Rx) is not in the memory 201, as determined at 409, the controller 200 may simply enable (at 410) the ID capability for the drug infusion device 102 to receive and store the auto program 113 (AP). This capability may be enabled in any appropriate manner, such as setting a flag indicating that the auto program receiving capability is enabled, running a subroutine specifically for handling the auto program 113 when it is received, ending a subroutine specifically designed to 15 reject the auto program 113 when it is received, etc. In general, the enabling at 410 may be considered as opening a window of time (a "receive auto program window of opportunity") during which the auto program 113 may be received and stored in the memory 201. This period of time may be made as long as possible or practical by enabling the receive auto program window as early in the process flow as possible. In some embodiments, the window of opportunity is opened after the clinical care area 106 has been selected or the identifier thereof has been transmitted to the server 103. In some embodiments, the window of opportunity is opened prior to receiving confirmation that the drug and concentration in the drug vial 215 is correct. In some embodiments the window of opportunity is opened prior to the vial 215 being inserted into the receptacle space 209. At any point after enabling the capability for the drug infusion device 102 to receive and store the auto program 113, when the auto program 113 is received through the communication engine 202, it is stored in the memory 201.

In some embodiments, it is desirable to have inserted the drug vial 215 into its proper insertion position in the receptacle space 209 by this point. Therefore, the controller 200 may check (at 411) for an error associated with the drug vial 215, which may be determined, for example, based on signals or data from the vial scanner 206 or the vial presence sensor 207. For example, the scanned vial drug identifiers (e.g., drug name and drug concentration) can be compared with the drug library 218. In another embodiment, if the infusion program 217 is present in the memory 201, then scanned vial drug identifiers (e.g., drug name and drug concentration) can be compared with the corresponding program drug identifiers in the infusion program 217 to determine whether the correct drug vial 215 has been inserted. The presence of an incorrect drug vial 215 may thus be a vial error in this situation. In some embodiments, the lack of the drug vial 215, as indicated by the signal from the vial presence sensor 207, may also indicate a vial error.

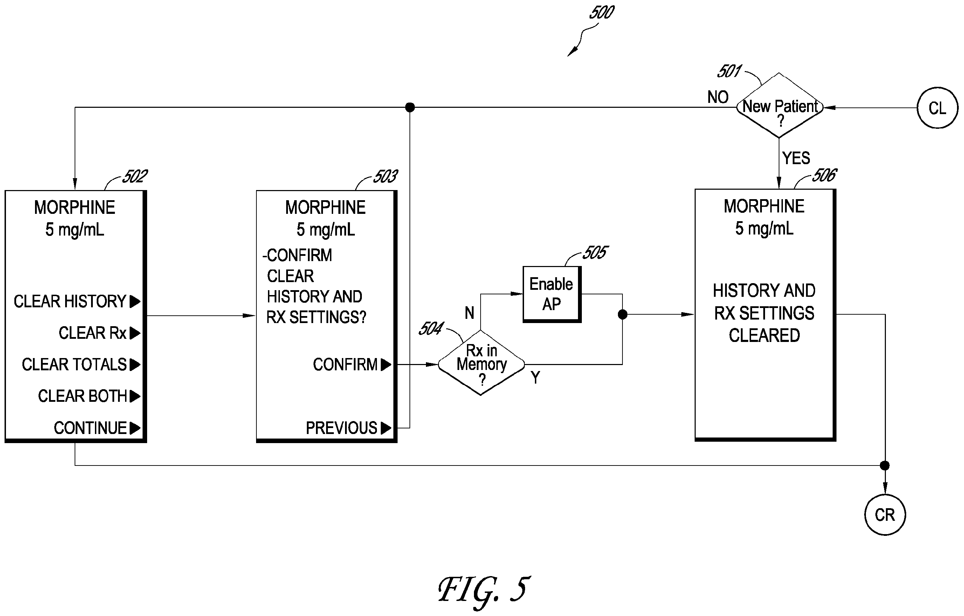

If there is a vial error, as determined at 411, then an appropriate vial error handling subroutine (at 412) may handle it. For example, the clinician may be prompted to insert the correct drug vial 215 or simply check to make sure the drug vial 215 is seated within the receptacle space 209 correctly. Alternatively, the clinician may be able to cancel out of the vial error handling subroutine in order to proceed to the portion of the process flow in which the infusion program 217, if present, may be cleared. In other embodiments, e.g., when the clinician plans to insert a different drug vial 215 with a different infusion program 217, the lack of the drug vial 215 at this point might not be a vial error, thereby allowing the clinician an opportunity to clear the preexisting infusion program 217 without having to insert the drug vial 215, be told that it does not match the preexisting infusion program 217, and cancel out of the vial error handling subroutine in order to proceed. Upon returning from the vial error handling subroutine (at 412) or if there was no vial error (at 411), the process flow proceeds to the next portion (designated herein as "Cl.," FIG. 5) for clearing the infusion program 217 if it is so desired.

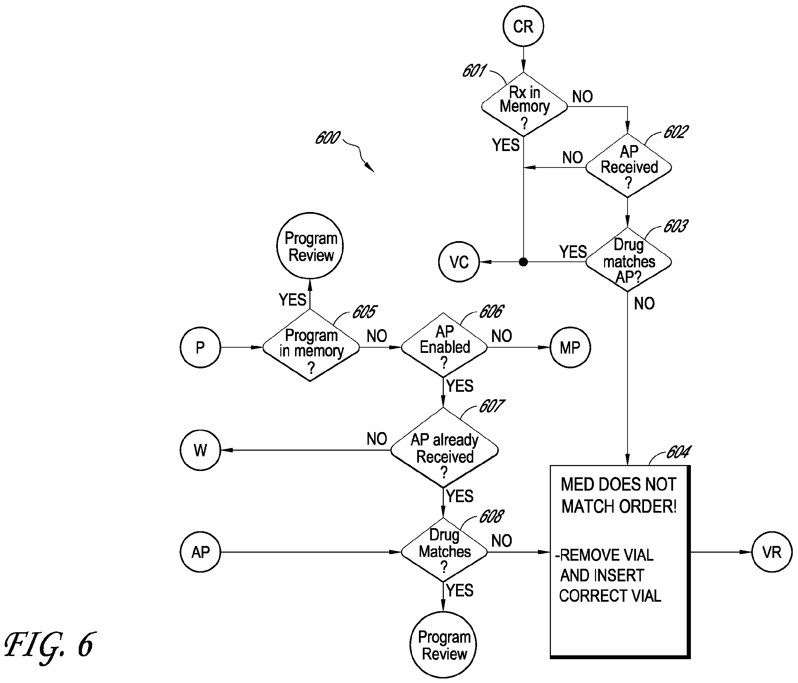

FIG. 5 shows a portion 500 of the example process flow that is generally for clearing the preexisting infusion program 217, if it is desired to do so. Thus, query 501 determines whether the infusion program 217 is already cleared, e.g., when the patient 105 is new, the process flow has been reset, etc. If not, i.e., if the infusion program 217 is present in the memory 201, then a menu screen 502 is presented to prompt the user to clear various data or to "continue" to the next portion (designated herein as "CR," FIG. 6), which handles branching between various other portions of the process flow. Additionally, the user may be presented with additional information, such as the drug name and drug concentration. The data that may be cleared generally includes the infusion program 217 ("clear Rx"), the log data ("history"), or just the totals of each ("clear totals"). Generally, only two of the three above items are displayed at anyone time, so another option to "clear 10 both" is displayed for selection. When the user selects one of these menu options for clearing data, a menu screen 503 is presented to the user to prompt the user to "confirm" the selection of data to be cleared or to return to the "previous" menu screen 502 to make a different selection.

If the infusion program 217 was cleared by the selections at 502 and 503, then it is possible at this point to open or enable the receive auto program (AP) window, described above. Therefore, query 504 checks whether the infusion program 217 ("Rx") is in the memory 201, and if not, the capability for the drug infusion device 102 to receive and store the auto program (AP) 113 is enabled at 505. In either case, a display screen 506 is presented to confirm to the user which data has been cleared. The display screen 506 may timeout after a period of time, or the user may make a selection on the keypad 203, to cause the process flow to continue to the next portion ("CR").

FIG. 6 shows a portion 600 of the example process flow that is generally for handling branching between various portions of the process flow for waiting for the auto program 113 to be received, confirming that the drug vial 215 is correct and matches what is specified in the auto program 113, optionally manually entering parameters for the infusion program 217 or bypassing the auto programming feature altogether, reviewing the infusion program 217 (or the auto program 113 as the infusion program 217), and/or triggering a reset of the process flow. At this point, the user has either confirmed the usage of the preexisting infusion program 217 or this data has been cleared from the memory 201.

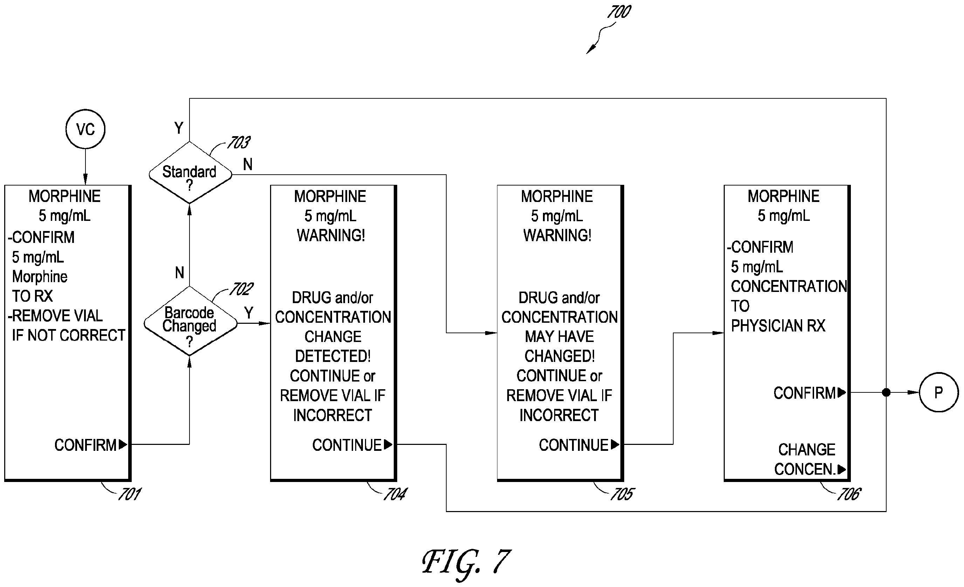

If the infusion program 217 has been cleared from the memory 201, then the receive auto program (AP) window will have been enabled, and the auto program 113 may have already been received. Therefore, query 601 determines whether an infusion program 217 ("Rx") is in the memory 201. If not, then query 602 determines whether the auto program (AP) 113 has been received. If so, and if the drug vial 215 is present, then query 603 determines whether the vial drug identifiers as determined by the vial scanner 206, external scanner 212 or a scanner in the handheld device 111 match corresponding program drug identifiers in the auto program 113. If not, then a display screen 604 is presented to the user to inform the user that the medication in the vial 215 does not match the order (i.e., the auto program 113) and to prompt the user to remove the drug vial 215 and insert a correct drug vial 215. When the vial scanner 206 makes the determination, the user can be assured that the correct drug vial 215 was actually inserted in the drug infusion device 102. When the user removes the drug vial 215, the vial reset function ("VR") is triggered, thereby resetting the process flow to the point described above. All alternative determinations at queries 601,602 and 603 (i.e., the infusion program 217 is in the memory 201, the auto program 113 has not been received, or the vial drug identifiers match the auto program 113) result in proceeding to the next portion (designated herein as "VC" FIG. 7), which prompts the user to confirm that the vial is correct.

FIG. 7 shows a portion 700 of the example process flow (branching from within portion 600) that is generally for prompting the user to make a series of manual confirmations that the drug vial 215 is correct. At display screen 701, the user is prompted to "confirm" whether a name of the drug and a concentration of the drug for the drug vial 215 are correct, i.e., match the drug name and concentration specified in the order or prescription ("Rx"). To present the display screen 701, the controller 200 uses the vial drug identifier scanned from the drug vial 215 to look up or read the name and concentration of the drug from the drug library 218. A confirmation received by the controller 200, thus, indicates that the drug vial 215 and the vial drug identifier are correct for the prescription. The display screen 701 instructs the user to remove the drug vial 215 if it is not correct, thereby triggering a vial reset function, as described above.

If the user selects to "confirm" that the drug vial 215 is correct, then additional checks are performed based on the vial drug identifiers determined from the vial scanner 206. For example, if the barcode on the drug vial 215 has not changed (assuming a barcode for the previous drug vial or the infusion program 217 is present in the memory 201), as determined at 702, and the barcode is a standard one (rather than a custom barcode for a vial custom filled and labeled by the customer), as determined at 703, then the process flow may proceed back to the process flow portion 600 (FIG. 6) at a point designated herein as "P."

On the other hand, if the barcode has changed, as determined at 702, then a display screen 704 is presented to the user with an extra warning that the drug or the concentration or both has been detected to have changed. The user is instructed again to remove the drug vial 215 if it is incorrect, thereby triggering the vial reset function, described above. However, if the user determines that the drug vial 215 is in fact correct, then the user may select "continue," and the process flow may proceed back to the process flow portion 600 (FIG. 6) at the point designated herein as "P."

Alternatively, if the barcode on the drug vial 215 is not a standard one, but a custom vial/barcode, as determined at 703, then a display screen 705 is presented to the user to warn that the drug or the concentration or both may have changed, since the same custom barcode could potentially be used in different situations. The user is instructed again to remove the drug vial 215 if it is incorrect, thereby triggering the vial reset function, described above.

However, if the user determines that the drug vial 215 is in fact correct, then the user may select "continue," in which case a display screen 706 is presented to the user to once again check to be sure that the concentration does in fact match the order or the doctor's prescription ("physician Rx"). If the vial drug concentration is correct, but the program drug concentration parameter in the infusion program 217 is wrong, then the user may select "change concentration" to receive a menu screen (not shown) to correct the infusion program 217. On the other hand, if the user once again determines that the drug vial 215 is in fact correct, then the user may select "confirm," and the process flow may proceed back to the process flow portion 600 (FIG. 6) at the point designated herein as "P."

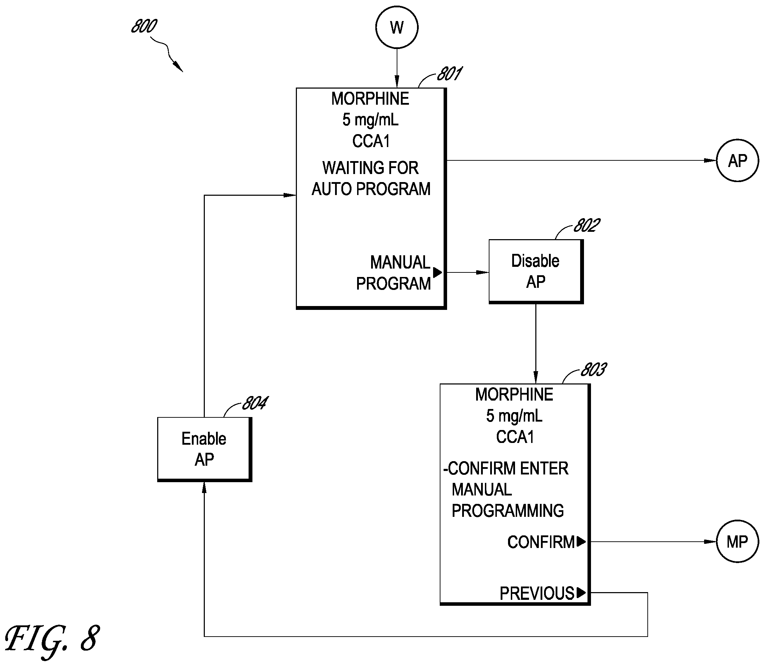

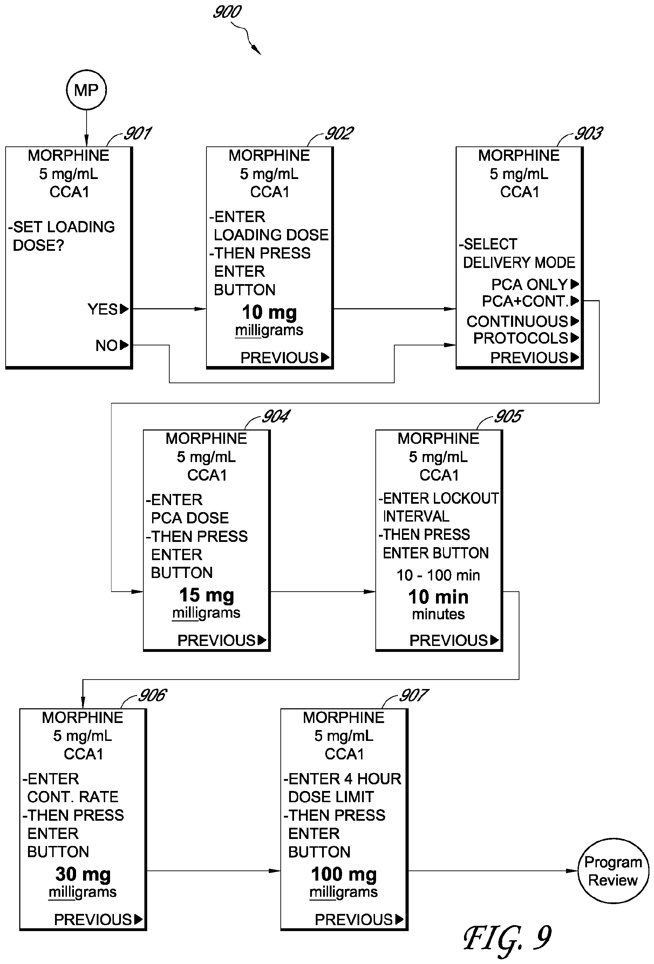

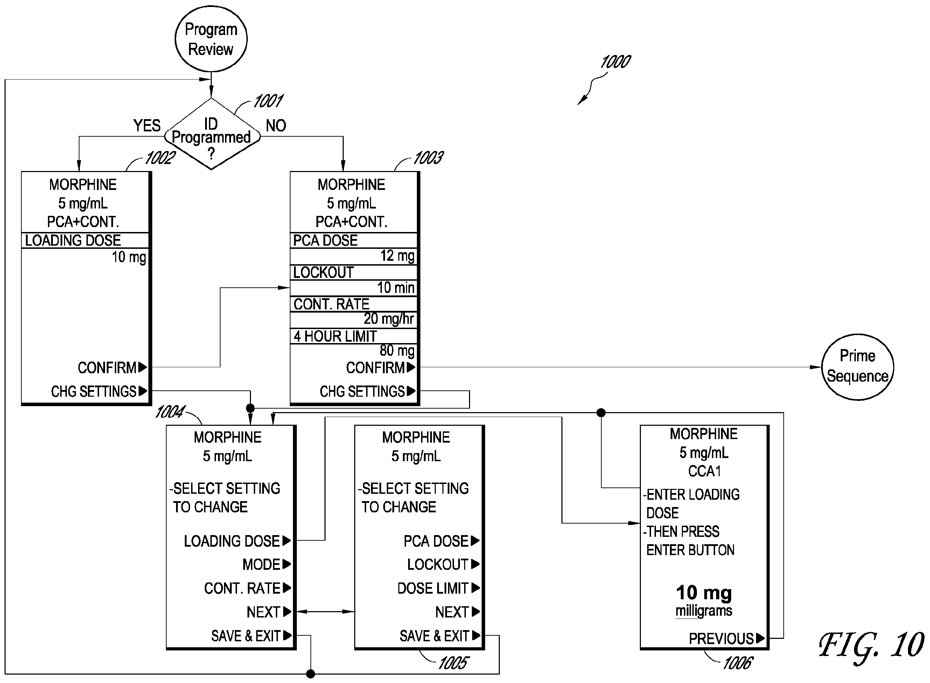

Referring back to FIG. 6, upon returning from the process flow portion 700 at the point designated as "P," if the infusion program 217 or the auto program 113 is in the memory 201, as determined at 605, then the process flow may proceed to a "program review" (FIG. 10, below). On the other hand, if neither the infusion program 217 nor the auto program 113 is in the memory 201, as determined at 605, and for some reason the receive auto program (AP) window has not been enabled, as determined at 606, then the process flow may proceed to a portion (designated herein as "MP") wherein the user can manually program the drug infusion device 102 (FIG. 9, below). If the receive auto program (AP) window has been enabled, as determined at 606, but the auto program 113 has not yet been received, as determined at 607, then the process flow may proceed to a portion (designated herein as "W") wherein the user is prompted (FIG. 8, below) to either wait for the auto program 113 or to select to proceed to the manual programming portion.

If the auto program 113 has been received, as determined at 607, and the vial drug identifiers, for example drug name and drug concentration from the drug library 218, match the corresponding program drug identifiers in the auto program 113, as determined at 608, then the process flow may proceed to the "program review" (FIG. 10, below). However, if the vial drug identifiers do not match the corresponding identifiers in the auto program 113, as determined at 608, then the display screen 604 is presented to the user to inform the user that the medication does not match the order (i.e., the auto program 113) and to prompt the user to remove the drug vial 215 and insert a correct drug vial 215. When the user removes the drug vial 215, the vial reset function ("VR") is triggered, thereby resetting the process flow to the point described above.

FIG. 8 shows a portion 800 of the example process flow (branching from within portion 600, as described above) that is generally for the user to wait for the auto program 113 or to select to proceed to the manual programming portion (FIG. 9, below). A display screen 801 is presented to the user to indicate that the drug infusion device 102 is waiting for the auto program 113 to be received from the medication management server 103. Additionally, the display screen 801 also provides an indication of the drug, the drug concentration, and the clinical care area 106 as a continued safety or security check for the benefit of the user. Furthermore, the display screen 801 also provides a menu option for the user to select to proceed to the manual programming portion (FIG. 9, below), e.g., in case the user learns that the auto program 113 is not going to be received. When the auto program 113 is received, the process flow automatically quits the display screen 801 and proceeds back to the process flow portion 600 (FIG. 6) at the point designated herein as "AP," so the process flow can proceed at query 608, as described above.

If the user decides not to wait for the auto program 113, then the user can select the "manual program" menu option. In this case, the receive auto program (AP) window is disabled (at 802), since it has been decided or assumed that the auto program 113 is not going to be used at this time, and it could potentially cause confusion between conflicting programs if the auto program 113 were to be received during the manual programming portion. Then a menu screen 803 is presented to the user to prompt the user either to "confirm" the selection to enter into the manual programming portion or to return to the "previous" display screen 801 to continue waiting for the auto program 113. If the user selects to "confirm" entering the manual programming portion, then the process flow may proceed to the portion (designated herein as "MP") wherein the user can manually program the drug infusion device 102 (FIG. 9, below). If the user selects to return to the "previous" display screen 801, then the receive auto program (AP) window is enabled again at 804 before the display screen 801 is presented.

In some embodiments, after the "manual program" is selected at display screen 801, the receive auto program (AP) window is not disabled until after the user selects to "confirm" entering the manual programming portion at the menu screen 803. In this case, if the auto program 113 is received while the menu screen 803 is presented, then the process flow may quit the menu screen 803 and either automatically proceed back to the process flow portion 600 (FIG. 6) at the point designated herein as "AP" or automatically present another menu screen (not shown) for the user to select whether to proceed back to the process flow portion 600 or onward to the manual programming portion.

FIG. 9 shows a portion 900 of the example process flow (branching from different process flow portions at points designated as "MP," as described above) that is generally for the user to follow a series of prompts to use the keypad 203 to manually enter the parameters for the infusion program 217. To do so, a menu screen 901 is presented to the user to select whether to set a "loading dose." The loading dose is a single dose of the drug given when the delivery of the drug to the patient 105 begins. The loading dose is thus typically some extra initial amount of the drug given to the patient 105, for example, to help get pain under control quickly. In some examples, the loading dose is not used, so the menu screen 901 is optional. If the user selects "yes" to enter a loading dose, a menu screen 902 is presented to prompt the user to enter the loading dose, e.g., to use numeric keys on the keypad 203 to enter a quantity in milligrams or some other appropriate units of measure. Then the user may press another key on the keypad 203, e.g., an "enter button," to proceed. Alternatively, the user may select "previous" to return to the menu screen 901. (Repeatedly selecting the "previous" menu screen at any point in the process flow portion 900 may allow the user to back step all the way to the beginning of the process flow portion 900 at any time.)

After proceeding from menu screen 902 or selecting "no" at menu screen 901, a menu screen 903 is presented to prompt the user to select the delivery mode. Alternatively, this menu screen 903 may be the first menu screen for the manual programming portion if a loading dose is not an option. The delivery modes are typically a PCA mode in which the patient 105 can receive an as-needed single on-demand PCA dose of the drug (as described above), a basal or continuous mode in which the patient 105 receives a steady volume over a period of time at a given rate, or a combination PCA and continuous mode in which both options are enabled. Additionally, other modes may be variations on these modes. The user may also select certain predefined "protocols" associated with one or more of the various delivery modes. Alternatively, the user may select to return to the "previous" menu screen 901 or 902.

If the user selected one of the delivery modes that includes a PCA dose at menu screen 903, a menu screen 904 is presented to prompt the user to enter the quantity of the PCA dose as a mass quantity, e.g., in milligrams or some other appropriate units of measure. The controller 200 can convert this quantity into a volume or flow rate value based on the vial drug concentration parameter of the drug in the drug vial 215. After entering the quantity for the PCA dose, the user may select to proceed, e.g., by pressing the "enter button." Alternatively, the user may select to return to the "previous" menu screen 903.

After selecting the PCA dose at menu screen 904, a menu screen 905 is presented to prompt the user to enter a "lockout interval" time period. The lockout interval time period is a minimum time period that must pass after the patient 105 triggers a PCA dose and before the patient 105 can trigger another PCA dose. After entering the time for the lockout interval, the user may select to proceed, e.g., by pressing the "enter button." Alternatively, the user may select to return to the "previous" menu screen 904.

If the user selected at menu screen 903 one of the delivery modes that includes a continuous drug delivery, a menu screen 906 is presented to prompt the user to enter a quantity for the rate at which the drug is to be continuously delivered. This quantity is typically a mass per time quantity (e.g., in milligrams per hour) or simply a mass quantity (e.g., with the time period assumed or predefined). After entering the quantity for the continuous rate, the user may select to proceed, e.g., by pressing the "enter button." Alternatively, the user may select to return to the "previous" menu screen 905.

Additionally, a menu screen 907 is presented to prompt the user to enter a maximum "dose limit" quantity of the drug that the patient 105 is permitted to receive over a designated time period, for example but not limited to one, four, eight, twelve or twenty-four hours. This maximum quantity value may further limit the amount of the PCA dose that the patient 105 can self-administer. After entering the quantity for the maximum dose limit, the user may select to proceed to the "program review" (FIG. 10, below), e.g., by pressing the "enter button." Alternatively, the user may select to return to the "previous" menu screen 906. Alternatively, the option can be provided for the user to select "no dose limit" of the sort described in this paragraph.

FIG. 10 shows a portion 1000 of the example process flow (branching from different process flow portions at points designated as "program review," as described above) that is generally for the user to review the infusion program 217 (as defined by the auto program 113 or as manually programmed by the user, clinician or nurse 110) before starting the actual delivery of the drug to the patient 105. If the loading dose has been set, as determined at 1001, a display screen 1002 is presented to the user to show the general parameters of the infusion program 217 (e.g., type of drug, concentration of drug in the drug vial 215, and delivery mode), as well as the specific loading dose quantity, described above. The user is also prompted to "confirm" the quantity or to change one or more of the settings.

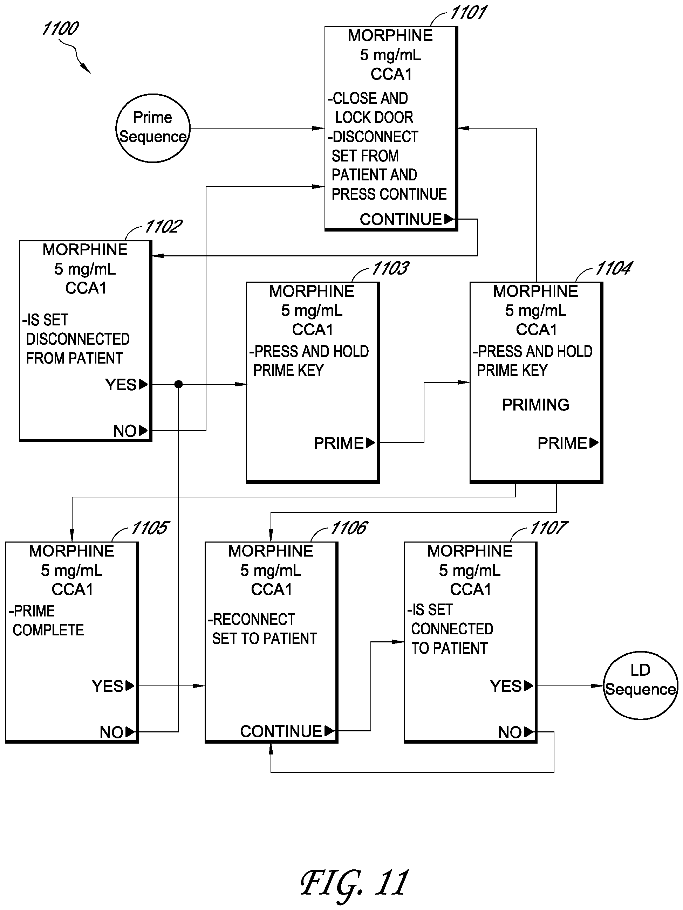

If the user confirms the loading dose quantity, or if no loading dose was set (as determined at 1001), a display screen 1003 is presented to the user to show the general parameters of the infusion program 217 (e.g., type of drug, concentration of drug in the drug vial 215, and delivery mode), as well as the specific quantities for the PCA dose quantity, the lockout interval time period, the continuous delivery rate quantity, and the dose limit quantity as appropriate. The user is also prompted to "confirm" the quantities or to change one or more of the settings. (In some examples, the menu screens 1002 and 1003 may be combined into one menu screen.) If the user selects "confirm" at menu screen 1003, then the parameters of the infusion program 217 or the auto program 113 have been confirmed, and the process flow can proceed to a "prime sequence" (FIG. 11, below).

If the user selects to change one or more of the settings (i.e., program parameters) at the menu screen 1002 or 1003, then a menu screen 1004 or 1005 is presented to prompt the user to select a setting to change. Not all of the settings may fit on a single menu screen, so the two menu screens 1004 and 1005 are used, and the user may switch between them by selecting "next." Each of the various programmed settings is presented on one of the menu screens 1004 or 1005. When the user selects one of the settings (e.g., loading dose, delivery mode, PCA dose, lockout interval, continuous delivery rate, or maximum dose limit), another menu screen (e.g., similar to menu screen 902,903,904,905, 906, or 907, respectively) is presented with which the user can change the quantity or value for that setting.

One example for these menu screens (menu screen 1006) is shown in FIG. 10. This example is for changing the loading dose setting, similar to the menu screen 902 (FIG. 9, above), and illustrates that when the user presses the "enter button" after entering a quantity or selects "previous" to cancel out of making a change, the menu screen 1004 or 1005 at which the user had selected to make the change is again presented.

In this manner, the user can continue to cycle or step through the menu screens 1004 and 1005 and the menu screens similar to 902, 903, 904, 905, 906, and 907 to make any necessary changes. When all necessary changes have been made, the user may select "save & exit" to return to query 1001 and menu screens 1002 and 1003 to review the current state of the various parameters or settings. The user may repeat this review process (1001-1005) until satisfied that the parameters are correct and then select "confirm" at both menu screens 1002 and 1003 to exit out of the program review and proceed to the prime sequence (FIG. 11).

In some embodiments, some variations of the program review may be different when doing an initial manual programming compared to when the auto program 113 is already in the memory 201 as the infusion program 217. For example, the "change settings" option may be available only when the auto program 113 or the infusion program 217 is already in the memory 201. Alternatively, the "previous" option may be available only when doing an initial manual programming.

FIG. 11 shows a portion 1100 ("prime sequence") of the example process flow (typically branching from the program review process flow portion 1000, as described above) that is generally for the user to ensure that the plumbing components such as the catheter 114 and the drug administration set 115 attached to the patient 105 and the vial 215 are properly primed with the drug, or purged of air bubbles. During the prime sequence, the pump unit 208 will flow a small quantity of the drug through the various components thereof. It is generally desirable, however, not to have this quantity of the drug delivered to the patient 105, because it could possibly result in an over-dosing of the patient 105. Therefore, a display screen 1101 is presented to the user to prompt the user to make sure that the drug infusion device 102 is disconnected from the patient 105 at this point. Additionally, for safety and security purposes, the user is also prompted to close and lock the door 210 of the receptacle space 209.

The controller 200 can determine whether the door is properly closed and/or locked by the signal from the door sensor 221 and can refuse to proceed if the door 210 is not properly closed and/or locked. However, the controller 200 cannot independently determine whether the drug infusion device 102 is disconnected from the patient 105, so when the user selects "continue" in order to proceed, a display screen 1102 is presented as a safety and security check to prompt the user to confirm that the drug infusion device 102 is indeed disconnected from the patient. In other words, the controller 200 prompts the clinician to confirm that the drug administration set 115 is not connected to the patient 105, or to the catheter 114. Selecting "no" causes the process flow to return to the menu screen 1101.

When the user selects "yes" at 1102, a menu screen 1103 is presented to prompt the user to "press and hold the prime key" on the keypad 203. When the user presses the prime key, a menu screen 1104 may be presented, which is similar to the menu screen 1103, but indicates that the drug infusion device 102 is performing the "priming." If the prime key is released before the prime sequence is complete, e.g., before a sufficient amount of the drug has been passed through the pump unit 208 to completely prime the drug administration set 115, a menu screen 1105 may be presented to prompt the user to confirm whether the prime is complete. If the user selects "no," then the process flow returns to the menu screen 1103 to prompt the user to continue to press and hold the prime key.

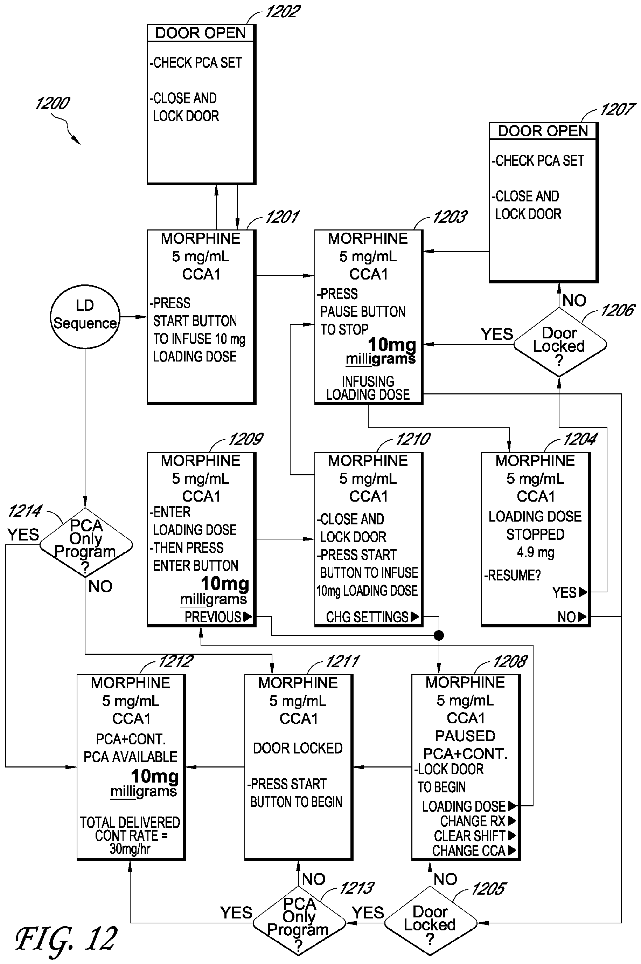

However, if the user selects "yes" at 1105, or if the controller 200 detects that the prime has completed, then a menu screen 1106 is presented to prompt the user to connect the drug infusion device 102 via the drug administration set 115 to the patient 105. When the user selects "continue" in order to proceed, a menu screen 1107 is presented to prompt the user to confirm whether the drug infusion device 102 has been connected to the patient 105. If the user selects "no," then the process flow returns to menu screen 1106 to again prompt the user to connect the drug administration set 115 to the patient 105. If the user selects "yes" at 1106, then the process flow proceeds to an "LD sequence" where, if an optional Loading Dose was specified, the device 102 can begin infusing, or delivering, the drug (FIG. 12, below).

FIG. 12 shows a portion 1200 (that defines the optional Loading Dose sequence and the infusion sequence) of the example process flow (typically branching from the prime sequence process flow portion 1100, as described above) that is generally for performing the drug delivery in accordance with the infusion program 217 or auto program 113. If the loading dose was included in the infusion program 217 or auto program 113, then a menu screen 1201 is presented to prompt the user to press a "start button" on the keypad 203 to cause the infusion of the loading dose, described above. If the door 210 is unlocked or opened at this point, then a menu screen 1202 is presented to indicate to the user that the door is open and to prompt the user to close and lock the door 210. Additionally, the menu screen 1202 prompts the user to check the drug administration set 115 or "PCA set," because the PCA function typically requires a special tube set and injector that is inserted into the drug vial 215, and these components may need to be checked to make sure that they have not come loose, and that a conventional slide clamp (not shown) has been closed whenever the door 210 is open to avoid unintended infusion or flow. When the door 210 is properly closed and locked, the slide clamp can be released or opened and process flow returns to the menu screen 1201.

When the user presses (and releases) the start button at 1201, a menu screen 1203 is presented to the user to indicate that the drug infusion device 102 is infusing the loading dose. The menu screen 1203 may also indicate the loading dose quantity. Additionally, the menu screen 1203 may indicate that the loading dose infusion can be paused or stopped by pressing a "pause button" on the keypad 203.

If the pause button is pressed or the door 210 is opened at 1203 before the loading dose delivery is completed, a menu screen 1204 is presented to indicate to the user that the loading dose has been stopped and either the remaining quantity of the loading dose to be delivered or the quantity that has been delivered. The menu screen 1204 also prompts the user to select whether to resume the loading dose. If the user selects "no" at 1204, then delivery of the remainder of the loading dose is cancelled and then the process flow proceeds to query 1205 to continue with the remainder of the program. If the user selects "yes" at 1204, the process flow proceeds to a query 1206 to check whether the door 210 is properly closed and locked. If not, then a menu screen 1207 (similar to 1202) is presented to indicate to the user that the door is open and to prompt the user to close and lock the door 210 and to check the "PCA set" (the drug administration set 115) as above. If the door is closed and locked (as determined at 1206 or as instructed at 1207), the process flow returns to the menu screen 1203 to indicate that the drug infusion device 102 is infusing the loading dose, as above. When the loading dose infusion at 1203 completes, the process flow proceeds to the query 1206 to check whether the door 210 is properly closed and locked, as above.

At the query 1205, i.e., after the loading dose infusion has completed (at 1203) or been canceled (by selecting "no" at 1204), the controller 200 determines whether the door 210 is closed and locked. If not, a menu screen 1208 is presented to indicate that the infusion program 217 has been paused. 15 The menu screen 1208 also instructs the user to close and lock the door 210 to begin the continuous and/or PCA portion of the infusion program 217. Additionally, at the menu screen 1208, the user is presented with another opportunity to change various parameters before beginning the continuous and/or PCA portion. To change the loading dose, the user may select "loading dose," and a menu screen 1209 (similar to 902) will be presented. If the user selects "previous" at 1209, the process flow may return to the menu screen 1208. If the user presses the "enter button" at 1209, a menu screen 1210 is presented to prompt the user to close and lock the door 210 and to press the "start button" again to begin infusing the loading dose at the menu screen 1203, as described above. However, if the user selects to change any of the settings at 1210, then the process flow may return to the menu screen 1208.

At the menu screen 1208, to change the prescription, i.e., the delivery mode and/or the mode parameters of the infusion program 207, the user may 30 select "change Rx," and menu screens similar to 903-907 will be presented in order. To clear shift totals of the amount infused, PCA demands attempted, loading dose, 4 hour dose, etc., the clinician may push the button associated with "clear shift". This may cause the totals to be reset to zero. To change the clinical care area 106 in the context of the work flow of FIG. 12, the user may select "change CCA" at 1208, and decisions and menu screens similar to 405 through 408 will be presented. The work flow permits the user to select a new CCA after being warned of the program clearing consequence of such a change, confirm the CCA change, and then the infuser 102 may receive a new auto program 113 or be manually programmed by the user 110. Unless the CCA was changed, the process flow may return to the menu screen 1208 to wait for the user to close and lock the door 210.

At the menu screen 1208, when the user closes and locks the door 210, a menu screen 1211 is presented to indicate to the user that the door 210 has been properly closed and locked. The menu screen 1211 also instructs the user to press the "start button" to begin the part of the infusion program 217 after the loading dose part, described above.

At the menu screen 1211, when the user presses the "start button," a menu screen 1212 is presented to indicate to the user that the drug is being delivered, the delivery mode, whether a PCA dose is currently available, the total quantity of the drug that has been delivered so far, and the continuous delivery rate.

Additionally, if the door 210 is determined to be closed and locked at 1205, then a query 1213 determines whether the infusion program 217 includes only a PCA mode. If so, then the process flow proceeds to the menu screen 1212, as above. However, if the infusion program 217 does not include only a PCA mode, as determined at 1213, the process flow proceeds to the menu screen 1211, as above.

Furthermore, if the loading dose was not included in the infusion program 217, then instead of starting at the menu screen 1201, as described above, the process flow portion 1200 proceeds to query 1214 to determine whether the infusion program 217 includes only a PCA mode. If so, then the process flow proceeds to the menu screen 1212, as above. However, if the infusion program 217 does not include only a PCA mode, as determined at 1214, the process flow proceeds to the menu screen 1211, as above.

In some embodiments, as explained above, the improved process for configuring and controlling the drug infusion device 102 includes various operation and control features that render the drug infusion device 102 capable of being programmed, set up or configured relatively quickly, so that the drug infusion device 102 can begin delivering the drug with very little setup time or with a minimum of human operator intervention. In different situations (e.g., depending on whether the patient is new, the clinical care area 106 has changed, the infusion program 217 is in the memory 201, the loading dose is included, etc.), the overall process takes a different path through the various portions of the process shown in FIGS. 3-12. In some situations, some portions of the process (e.g., FIGS. 8 and 9) are skipped altogether. An example effect of the improved overall process is that the most common situation (or situations) results in an optimized/fastest setup time and/or the least operator intervention/action. Consequently, it is more often the case that the patient can begin receiving the necessary drug more quickly, the operator's time is more efficiently used, and the chances for human error are reduced, among other potential advantages or improvements.

For example, a common situation occurs when the drug infusion device 102 has been allocated for use with a new patient (and a standard drug) after having been left idle for a few hours, and the correct auto program 113 is received relatively early during the window of opportunity. In this case, after the start-up and power-on self-test portion 300 (FIG. 3) of the overall process flow, the process passes through 401,404, optionally 405 and 406, and 408-411 of the process flow portion 400 (FIG. 4). In particular, it is sometimes determined at 401 that the drug infusion device 102 should be completely reset, since it has been idle for at least a predetermined period of time such as a few hours. Thus, any stored infusion program 217 is deleted at this point, so the "receive auto program window of opportunity" can be opened as soon as possible. Additionally, after the clinical care area 106 is selected or confirmed (404-405), an identifier for the clinical care area 106 is transmitted to the server 103 (at 408). Thus, the "receive auto program window of opportunity" is opened at 410, which can be before or after transmitting the identifier to the server 103. In some embodiments, the "receive auto program window of opportunity" is opened after selection of the clinical care area 106 at 404-405.

In the process flow portion 500 (FIG. 5), if a new patient as determined at 501 (meaning that the infusion program 217 has already been deleted), steps 502-505 are skipped, and the display screen 506 is presented to confirm to the operator that the data has been cleared. Then, in the process flow portion 600 (FIG. 6), assuming that the auto program 113 has been received, and that the drug vial 215 has been properly inserted into the receptacle space 209, the process passes through 601-603 to the process portion 700. At display screen 701, the controller 200 uses the drug identifier (scanned from the barcode of the drug vial 215) to look up or read the drug name and drug concentration from the drug library 218, and present this information to the operator, so the operator can confirm that the drug vial 215 (and, thus, the drug identifier) is correct. The controller 200 receives an indication of this confirmation. The drug infusion device 102 determines (at 702) that the barcode of the drug vial 215 has not changed, since there is no previous barcode to compare the new barcode to, due to the settings having been cleared. Since the drug is standard, as determined at 703, the process returns to the process flow portion 600 to branch (at 605) to the program review process flow portion 1000 (FIG. 10). If the auto program 113 has not been received yet, however, the process branches (at 607) to the waiting process flow portion 800 before returning to branch (through 608) to the program review process flow portion 1000.

At the display screens 1002 and 1003, the operator confirms the loading dose (if set) and the rest of the parameters of the infusion program 217, respectively, are correct. The prime sequence process flow portion 1100 (FIG. 11), followed by the process flow portion 1200 (FIG. 12), are then performed as described above.

While the specification has been described in detail with respect to specific embodiments of the present invention, it will be appreciated that those skilled in the art, upon attaining an understanding of the foregoing, may readily conceive of alterations to, variations of, and equivalents to these embodiments. These and other modifications and variations to the present invention may be practiced by those skilled in the art, without departing from the scope of the present invention, which is more particularly set forth in the appended claims.

## Claims

1. A method comprising:
determining, by a controller (200) of a drug infusion device (102), whether an infusion program (107, 217) is stored within a memory (201) of the drug infusion device, the drug infusion device being capable of being connected to a patient and delivering a drug to the patient in accordance with the infusion program under control of the controller after configuration of the drug infusion device according to parameters specified by the infusion program;
if the infusion program is stored within the memory, prompting, by the controller with a first series of prompts, a user to confirm usage of the infusion program, to make a confirmation of a drug identifier, and to review the configuration of the drug infusion device according to the parameters specified by the infusion program;
if the infusion program is not stored within the memory and a clinical care area (106) in which the drug infusion device is located or to be used has been identified by a clinical care area identifier, enabling, by the controller, a capability for the drug infusion device to receive and store in the memory of the drug infusion device an auto program (113) with which the drug infusion device can configure itself without intervention by the user according to parameters specified by the auto program as the infusion program;
wherein said enabling opens a window of time to receive and store the auto program for the identified clinical care area,
if the auto program is not received after the capability for the drug infusion device to receive and store the auto program is enabled, waiting, by the controller, for receipt of the auto program or for a selection by the user to manually enter the infusion program; and
when the controller begins waiting for receipt of the auto program or for a selection by the user to manually enter the infusion program, displaying, by the controller, a menu screen indicating that the drug infusion device is waiting for the auto program; and wherein if the auto program is received i) while the capability for the drug infusion device to receive and store the auto program is enabled and ii) before the controller begins waiting for receipt of the auto program or for a selection by the user to manually enter the infusion program, then the menu screen indicating that the drug infusion device is waiting for the auto program is not displayed,
wherein the capability for the drug infusion device to receive and store the auto program is not enabled by the controller unless an infusion program previously stored in memory is cleared or completed.

2. The method of claim 1, further comprising:
performing, by the controller, a power-on self-test of the drug infusion device;
receiving the container of the drug into a lockable receptacle space (209) within a housing (213) of the drug infusion device, the controller being capable of receiving a reset signal indicating that the container of the drug has been removed from the lockable receptacle space;
if the reset signal is received by the controller after the performing of the power-on self-test, stopping, by the controller, the method during any step therein and resetting, by the controller, the method to a point after the performing of the power-on self-test; and if the reset signal is not received by the controller, performing the method without resetting the method.

3. The method of claim 1, wherein:
the prompting, by the controller with the first series of prompts, further comprises:
prompting the user to select one of confirm usage of the infusion program, and clear the infusion program; and
in response to confirm usage of the infusion program being selected, prompting the user to make the confirmation of the drug identifier and to review the configuration of the drug infusion device according to the parameters specified by the infusion program; and
the method further comprises:
if clear the infusion program is selected, enabling, by the controller, the capability for the drug infusion device to receive the auto program.

4. The method of claim 1, wherein:
the infusion program also specifies a program drug identifier; and the method further comprises:
receiving a drug vial (215) into a lockable receptacle space (209) within a housing (213) of the drug infusion device, the drug vial having indicia specifying a vial drug identifier;
determining, by the controller, the vial drug identifier directly from the indicia of the drug vial;
if the vial drug identifier matches the program drug identifier, performing, by the controller with the first series of prompts, the prompting of the user to review the configuration of the drug infusion device according to the parameters specified by the infusion program; and if one of the vial drug identifier does not match the program drug identifier, prompting, by the controller with a second series of prompts, the user to check the drug vial.

5. The method of claim 4 further comprising:
in response to removal of the drug vial from the lockable receptacle space, generating, by a vial presence sensor (207), a vial-reset signal;
receiving, by the controller, the vial-reset signal; and
in response to the vial-reset signal, resetting, by the controller, a process for the configuration of the drug infusion device.

6. An infusion device (102) comprising;
a memory (201), and
a controller (200) for determining whether an infusion program (107, 217) is stored within the memory of the drug infusion device, the drug infusion device being capable of being connected to a patient and delivering a drug to the patient in accordance with the infusion program under control of the controller after configuration of the drug infusion device according to parameters specified by the infusion program;
if the infusion program is stored within the memory, prompting, by the controller with a first series of prompts, a user to confirm usage of the infusion program, to make a confirmation of a drug identifier, and to review the configuration of the drug infusion device according to the parameters specified by the infusion program;
if the infusion program is not stored within the memory, and a clinical care area (106) in which the drug infusion device is located or to be used has been identified by a clinical care area identifier, enabling, by the controller, a capability for the drug infusion device to receive and store in the memory of the drug infusion device an auto program (113) with which the drug infusion device can configure itself without intervention by the user according to parameters specified by the auto program as the infusion program;
wherein said enabling opens a window of time to receive and store the auto program for the identified clinical care area,
if the auto program is not received after the capability for the drug infusion device to receive and store the auto program is enabled, waiting, by the controller, for receipt of the auto program or for a selection by the user to manually enter the infusion program; and
when the controller begins waiting for receipt of the auto program or for a selection by the user to manually enter the infusion program, displaying, by the controller, a menu screen indicating that the drug infusion device is waiting for the auto program; and wherein if the auto program is received i) while the capability for the drug infusion device to receive and store the auto program is enabled and ii) before the controller begins waiting for receipt of the auto program or for a selection by the user to manually enter the infusion program, then the menu screen indicating that the drug infusion device is waiting for the auto program is not displayed, wherein the capability for the drug infusion device to receive and store the auto program is not enabled by the controller unless an infusion program previously stored in memory is cleared or completed.

7. An infusion device according to claim 6 further comprising,
a communication engine (202) for communication with a medication management server (103).

8. An infusion device according to claim 7,
wherein the communication engine is configured to transmit and/or receive the auto program, infusion program, log files and messages.

9. An infusion device according to one of the preceding claims 6-8 further comprising,
a keypad (203) for the user to manually enter or confirm usage of the infusion program, or to make a confirmation of a drug identifier, and
a display (204) for displaying the prompts and the menu screen.

10. A medication administering system (100) comprising,
a medication management server (103) configured to generate an auto program (113) or route the auto program further to an infusion device (102), and
the infusion device, the infusion device comprises,
a memory (201), and
a controller (200) for determining whether an infusion program (107, 217) is stored within the memory of the drug infusion device, the drug infusion device being capable of being connected to a patient and delivering a drug to the patient in accordance with the infusion program under control of the controller after configuration of the drug infusion device according to parameters specified by the infusion program;
if the infusion program is stored within the memory, prompting, by the controller with a first series of prompts, a user to confirm usage of the infusion program, to make a confirmation of a drug identifier, and to review the configuration of the drug infusion device according to the parameters specified by the infusion program;
if the infusion program is not stored within the memory and a clinical care area (106) in which the drug infusion device is located or to be used has been identified by a clinical care area identifier, enabling, by the controller, a capability for the drug infusion device to receive and store in the memory of the drug infusion device the auto program with which the drug infusion device can configure itself without intervention by the user according to parameters specified by the auto program as the infusion program;
wherein said enabling opens a window of time to receive and store the auto program for the identified clinical care area,
if the auto program is not received after the capability for the drug infusion device to receive and store the auto program is enabled, waiting, by the controller, for receipt of the auto program or for a selection by the user to manually enter the infusion program; and
when the controller begins waiting for receipt of the auto program or for a selection by the user to manually enter the infusion program, displaying, by the controller, a menu screen indicating that the drug infusion device is waiting for the auto program; and wherein if the auto program is received i) while the capability for the drug infusion device to receive and store the auto program is enabled, and ii) before the controller begins waiting for receipt of the auto program or for a selection by the user to manually enter the infusion program, then the menu screen indicating that the drug infusion device is waiting for the auto program is not displayed, wherein the capability for the drug infusion device to receive and store the auto program is not enabled by the controller unless an infusion program previously stored in memory is cleared or completed.

11. A medication administering system according to claim 10 further comprising,
at least one additional of a computer, workstation, server or network communication device (104).

12. A medication administering system according to one of the preceding claims 10-11,
wherein the medication administering system further comprises a database (112).

13. A medication administering system according to claim 12,
wherein the medication management server is configured to generate the infusion program based on a prescription.

14. A medication administering system according to claim 12,
wherein the database comprises at least one of a drug data describing the various drugs that the drug infusion device may deliver to a patient, allowable parameters for programming the drug infusion device under various conditions or allowable parameters within various clinical care areas.

## Patentansprüche

1. Verfahren, umfassend:
Bestimmen, durch eine Steuereinheit (200) einer Arzneimittelinfusionsvorrichtung (102), ob ein Infusionsprogramm (107, 217) in einem Speicher (201) der Arzneimittelinfusionsvorrichtung gespeichert ist, wobei die Arzneimittelinfusionsvorrichtung in der Lage ist, mit einem Patienten verbunden zu werden und an einen Patienten ein Arzneimittel gemäß dem Infusionsprogramm unter Steuerung der Steuereinheit nach Konfiguration der Arzneimittelinfusionsvorrichtung gemäß durch das Infusionsprogramm festgelegten Parametern abzugeben;
wenn das Infusionsprogramm im Speicher gespeichert ist, Auffordern eines Benutzers durch die Steuereinheit mit einer ersten Serie von Aufforderungen, die Verwendung des Infusionsprogramms zu bestätigen, eine Bestätigung einer Arzneimittelkennung vorzunehmen und die Konfiguration der Arzneimittelinfusionsvorrichtung gemäß den durch das Infusionsprogramm festgelegten Parametern zu überprüfen;
wenn das Infusionsprogramm nicht im Speicher gespeichert ist und ein klinischer Versorgungsbereich (106), in dem sich die Arzneimittelinfusionsvorrichtung befindet oder verwendet werden soll, durch eine Kennung des klinischen Versorgungsbereichs identifiziert wurde, Aktivieren einer Fähigkeit für die Arzneimittelinfusionsvorrichtung durch die Steuereinheit, ein Autoprogramm (113) zu empfangen und im Speicher der Arzneimittelinfusionsvorrichtung zu speichern, mit dem sich die Arzneimittelinfusionsvorrichtung ohne Eingriff durch den Benutzer selbst gemäß Parametern konfigurieren kann, die durch das Autoprogramm als das Infusionsprogramm festgelegt sind;
wobei das Aktivieren ein Zeitfenster öffnet, um das Autoprogramm für den identifizierten klinischen Versorgungsbereich zu empfangen und zu speichern,
wenn das Autoprogramm nicht empfangen wird, nachdem die Fähigkeit für die Arzneimittelinfusionsvorrichtung, das Autoprogramm zu empfangen und zu speichern, aktiviert wurde, Warten durch die Steuereinheit auf den Empfang des Autoprogramms oder auf eine Auswahl durch den Benutzer, um das Infusionsprogramm manuell einzugeben; und
wenn die Steuereinheit beginnt, auf den Empfang des Autoprogramms oder auf eine Auswahl durch den Benutzer zur manuellen Eingabe des Infusionsprogramms zu warten, Anzeigen eines Menübildschirms durch die Steuereinheit, der anzeigt, dass die Arzneimittelinfusionsvorrichtung auf das Autoprogramm wartet; und wobei, wenn das Autoprogramm empfangen wird, i) während die Fähigkeit für die Arzneimittelinfusionsvorrichtung, das Autoprogramm zu empfangen und zu speichern, aktiviert ist und ii) bevor die Steuereinheit beginnt, auf den Empfang des Autoprogramms oder auf eine Auswahl durch den Benutzer zur manuellen Eingabe des Infusionsprogramms zu warten, der Menübildschirm, der anzeigt, dass die Arzneimittelinfusionsvorrichtung auf das Autoprogramm wartet, dann nicht angezeigt wird,
wobei die Fähigkeit für die Arzneimittelinfusionsvorrichtung, das Autoprogramm zu empfangen und zu speichern, von der Steuereinheit nicht aktiviert wird, solange ein zuvor in der Steuereinheit gespeichertes Infusionsprogramm nicht gelöscht oder abgeschlossen ist.

2. Verfahren nach Anspruch 1, weiter umfassend:
Durchführen eines Einschalt-Selbsttests der Arzneimittelinfusionsvorrichtung durch die Steuereinheit;
Aufnehmen des Behältnisses des Arzneimittels in einen verschließbaren Aufnahmeraum (209) innerhalb eines Gehäuses (213) der Arzneimittelinfusionsvorrichtung, wobei die Steuereinheit in der Lage ist, ein Rücksetzsignal zu empfangen, das anzeigt, dass das Behältnis des Arzneimittels aus dem verschließbaren Aufnahmeraum entfernt worden ist;
wenn das Rücksetzsignal von der Steuereinheit nach der Durchführung des Einschalt-Selbsttests empfangen wird, Anhalten des Verfahrens durch die Steuereinheit während eines beliebigen Schrittes darin und Zurücksetzen des Verfahrens durch die Steuereinheit auf einen Punkt nach der Durchführung des Einschalt-Selbsttests; und wenn das Rücksetzsignal nicht von der Steuereinheit empfangen wird, Durchführen des Verfahrens ohne Zurücksetzen des Verfahrens.

3. Verfahren nach Anspruch 1, wobei:
das Auffordern durch die Steuereinheit mit der ersten Serie von Aufforderungen weiter umfasst:
Auffordern des Benutzers zum Auswählen eines von: Bestätigen der Verwendung des Infusionsprogramms und Löschen des Infusionsprogramms; und
als Reaktion auf das Bestätigen der Verwendung des ausgewählten Infusionsprogramms, Auffordern des Benutzers, die Bestätigung der Arzneimittelkennung vorzunehmen und die Konfiguration der Arzneimittelinfusionsvorrichtung gemäß den durch das Infusionsprogramm festgelegten Parametern zu überprüfen; und
das Verfahren weiter umfasst:
wenn Löschen des Infusionsprogramms ausgewählt ist, Aktivieren der Fähigkeit für die Arzneimittelinfusionsvorrichtung durch die Steuereinheit, das Autoprogramm zu empfangen.

4. Verfahren nach Anspruch 1, wobei:
das Infusionsprogramm auch eine Programm-Arzneimittelkennung festlegt; und das Verfahren weiter umfasst:
Aufnehmen einer Arzneimittelampulle (215) in einen verschließbaren Aufnahmeraum (209) innerhalb eines Gehäuses (213) der Arzneimittelinfusionsvorrichtung, wobei die Arzneimittelampulle Markierungen aufweist, die eine Ampullen-Arzneimittelkennung festlegen;
Bestimmen der Ampullen-Arzneimittelkennung durch die Steuereinheit direkt anhand der Markierungen der Arzneimittelampulle;
wenn die Ampullen-Arzneimittelkennung mit der Programm-Arzneimittelkennung übereinstimmt, Durchführen, durch die Steuereinheit mit der ersten Serie von Aufforderungen, des Aufforderns des Benutzers, die Konfiguration der Arzneimittelinfusionsvorrichtung gemäß den durch das Infusionsprogramm festgelegten Parametern zu überprüfen; und wenn eine der Ampullen-Arzneimittelkennung nicht mit der Programm-Arzneimittelkennung übereinstimmt, Auffordern, durch die Steuereinheit mit einer zweiten Serie von Aufforderungen, des Benutzers, die Arzneimittelampulle zu kontrollieren.

5. Verfahren nach Anspruch 4, weiter umfassend:
als Reaktion auf das Entfernen der Arzneimittelampulle aus dem verschließbaren Aufnahmeraum, Erzeugen eines Ampullen-Rücksetzsignals durch einen Ampullen-Präsenzsensor (207);
Empfangen des Ampullen-Rücksetzsignals durch die Steuereinheit; und
als Reaktion auf das Ampullen-Rücksetzsignal, Zurücksetzen eines Prozesses zur Konfiguration der Arzneimittelinfusionsvorrichtung durch die Steuereinheit.

6. Infusionsvorrichtung (102), umfassend;
einen Speicher (201), und
eine Steuereinheit (200) zum Bestimmen, ob ein Infusionsprogramm (107, 217) in dem Speicher der Arzneimittelinfusionsvorrichtung gespeichert ist, wobei die Arzneimittelinfusionsvorrichtung in der Lage ist, mit einem Patienten verbunden zu werden und an einen Patienten ein Arzneimittel gemäß dem Infusionsprogramm unter der Steuerung der Steuereinheit nach Konfiguration der Arzneimittelinfusionsvorrichtung gemäß den durch das Infusionsprogramm festgelegten Parametern abzugeben;
wenn das Infusionsprogramm im Speicher gespeichert ist, Auffordern eines Benutzers durch die Steuereinheit mit einer ersten Serie von Aufforderungen, die Verwendung des Infusionsprogramms zu bestätigen, eine Bestätigung einer Arzneimittelkennung vorzunehmen und die Konfiguration der Arzneimittelinfusionsvorrichtung gemäß den durch das Infusionsprogramm festgelegten Parametern zu überprüfen;
wenn das Infusionsprogramm nicht im Speicher gespeichert ist und ein klinischer Versorgungsbereich (106), in dem sich die Arzneimittelinfusionsvorrichtung befindet oder verwendet werden soll, durch eine Kennung des klinischen Versorgungsbereichs identifiziert wurde, Aktivieren einer Fähigkeit für die Arzneimittelinfusionsvorrichtung durch die Steuereinheit, ein Autoprogramm (113) zu empfangen und im Speicher der Arzneimittelinfusionsvorrichtung zu speichern, mit dem sich die Arzneimittelinfusionsvorrichtung ohne Eingriff durch den Benutzer selbst gemäß Parametern konfigurieren kann, die durch das Autoprogramm als das Infusionsprogramm festgelegt sind;
wobei das Aktivieren ein Zeitfenster öffnet, um das Autoprogramm für den identifizierten klinischen Versorgungsbereich zu empfangen und zu speichern,
wenn das Autoprogramm nicht empfangen wird, nachdem die Fähigkeit für die Arzneimittelinfusionsvorrichtung, das Autoprogramm zu empfangen und zu speichern, aktiviert wurde, Warten durch die Steuereinheit auf den Empfang des Autoprogramms oder auf eine Auswahl durch den Benutzer, um das Infusionsprogramm manuell einzugeben; und
wenn die Steuereinheit beginnt, auf den Empfang des Autoprogramms oder auf eine Auswahl durch den Benutzer zur manuellen Eingabe des Infusionsprogramms zu warten, Anzeigen eines Menübildschirms durch die Steuereinheit, der anzeigt, dass die Arzneimittelinfusionsvorrichtung auf das Autoprogramm wartet; und wobei, wenn das Autoprogramm empfangen wird, i) während die Fähigkeit für die Arzneimittelinfusionsvorrichtung, das Autoprogramm zu empfangen und zu speichern, aktiviert ist und ii) bevor die Steuereinheit beginnt, auf den Empfang des Autoprogramms oder auf eine Auswahl durch den Benutzer zur manuellen Eingabe des Infusionsprogramms zu warten, der Menübildschirm, der anzeigt, dass die Arzneimittelinfusionsvorrichtung auf das Autoprogramm wartet, dann nicht angezeigt wird, wobei die Fähigkeit für die Arzneimittelinfusionsvorrichtung, das Autoprogramm zu empfangen und zu speichern, von der Steuereinheit nicht aktiviert wird, solange ein zuvor in der Steuereinheit gespeichertes Infusionsprogramm nicht gelöscht oder abgeschlossen ist.

7. Infusionsvorrichtung nach Anspruch 6, weiter umfassend,
eine Kommunikationsmaschine (202) zur Kommunikation mit einem Medikamentenverwaltungsserver (103).

8. Infusionsvorrichtung nach Anspruch 7,
wobei die Kommunikationsmaschine dazu konfiguriert ist, das Autoprogramm, das Infusionsprogramm, die Protokolldateien und die Meldungen zu senden und/oder zu empfangen.

9. Infusionsvorrichtung nach einem der vorstehenden Ansprüche 6-8, weiter umfassend,
ein Tastenfeld (203) für den Benutzer zum manuellen Eingeben oder Bestätigen der Verwendung des Infusionsprogramms oder zum Vornehmen einer Bestätigung einer Arzneimittelkennung, und
eine Anzeige (204) zum Anzeigen der Aufforderungen und des Menübildschirms.

10. Medikamentenverabreichungssystem (100), umfassend,
einen Medikamentenverwaltungsserver (103), der dazu konfiguriert ist, ein Autoprogramm (113) zu erzeugen oder das Autoprogramm weiter an eine Infusionsvorrichtung (102) zu leiten, und
die Infusionsvorrichtung, wobei die Infusionsvorrichtung umfasst,
einen Speicher (201), und
eine Steuereinheit (200) zum Bestimmen, ob ein Infusionsprogramm (107, 217) in dem Speicher der Arzneimittelinfusionsvorrichtung gespeichert ist, wobei die Arzneimittelinfusionsvorrichtung in der Lage ist, mit einem Patienten verbunden zu werden und an einen Patienten ein Arzneimittel gemäß dem Infusionsprogramm unter der Steuerung der Steuereinheit nach Konfiguration der Arzneimittelinfusionsvorrichtung gemäß den durch das Infusionsprogramm festgelegten Parametern abzugeben;
wenn das Infusionsprogramm im Speicher gespeichert ist, Auffordern eines Benutzers durch die Steuereinheit mit einer ersten Serie von Aufforderungen, die Verwendung des Infusionsprogramms zu bestätigen, eine Bestätigung einer Arzneimittelkennung vorzunehmen und die Konfiguration der Arzneimittelinfusionsvorrichtung gemäß den durch das Infusionsprogramm festgelegten Parametern zu überprüfen;
wenn das Infusionsprogramm nicht im Speicher gespeichert ist und ein klinischer Versorgungsbereich (106), in dem sich die Arzneimittelinfusionsvorrichtung befindet oder verwendet werden soll, durch eine Kennung des klinischen Versorgungsbereichs identifiziert worden ist, Aktivieren einer Fähigkeit für die Arzneimittelinfusionsvorrichtung durch die Steuereinheit, das Autoprogramm zu empfangen und im Speicher der Arzneimittelinfusionsvorrichtung zu speichern, mit dem sich die Arzneimittelinfusionsvorrichtung ohne Eingriff durch den Benutzer selbst gemäß Parametern konfigurieren kann, die durch das Autoprogramm als das Infusionsprogramm festgelegt sind;
wobei das Aktivieren ein Zeitfenster öffnet, um das Autoprogramm für den identifizierten klinischen Versorgungsbereich zu empfangen und zu speichern,
wenn das Autoprogramm nicht empfangen wird, nachdem die Fähigkeit für die Arzneimittelinfusionsvorrichtung, das Autoprogramm zu empfangen und zu speichern, aktiviert wurde, Warten durch die Steuereinheit auf den Empfang des Autoprogramms oder auf eine Auswahl durch den Benutzer, um das Infusionsprogramm manuell einzugeben; und
wenn die Steuereinheit beginnt, auf den Empfang des Autoprogramms oder auf eine Auswahl durch den Benutzer zur manuellen Eingabe des Infusionsprogramms zu warten, Anzeigen eines Menübildschirms durch die Steuereinheit, der anzeigt, dass die Arzneimittelinfusionsvorrichtung auf das Autoprogramm wartet; und wobei, wenn das Autoprogramm empfangen wird, i) während die Fähigkeit für die Arzneimittelinfusionsvorrichtung, das Autoprogramm zu empfangen und zu speichern, aktiviert ist und ii) bevor die Steuereinheit beginnt, auf den Empfang des Autoprogramms oder auf eine Auswahl durch den Benutzer zur manuellen Eingabe des Infusionsprogramms zu warten, der Menübildschirm, der anzeigt, dass die Arzneimittelinfusionsvorrichtung auf das Autoprogramm wartet, dann nicht angezeigt wird, wobei die Fähigkeit für die Arzneimittelinfusionsvorrichtung, das Autoprogramm zu empfangen und zu speichern, von der Steuereinheit nicht aktiviert wird, solange ein zuvor in der Steuereinheit gespeichertes Infusionsprogramm nicht gelöscht oder abgeschlossen ist.

11. Medikamentenverabreichungssystem nach Anspruch 10, weiter umfassend,
mindestens ein zusätzliches von Computer, Arbeitsstation, Server oder Netzwerkkommunikationsvorrichtung (104).

12. Medikamentenverabreichungssystem nach einem der vorstehenden Ansprüche 10-11,
wobei das Medikamentenverabreichungssystem weiter eine Datenbank (112) umfasst.

13. Medikamentenverabreichungssystem nach Anspruch 12,
wobei der Medikamentenverwaltungsserver dazu konfiguriert ist, das Infusionsprogramm auf Grundlage eines Rezepts zu erzeugen.

14. Medikamentenverabreichungssystem nach Anspruch 12,
wobei die Datenbank mindestens eines von Arzneimitteldaten umfasst, die die verschiedenen Arzneimittel beschreiben, die die Arzneimittelinfusionsvorrichtung an einen Patienten abgeben kann, zulässige Parameter zur Programmierung der Arzneimittelinfusionsvorrichtung unter verschiedenen Bedingungen oder zulässige Parameter innerhalb verschiedener klinischer Versorgungsbereiche.

## Revendications

1. Procédé comprenant :
la détermination, par un dispositif de commande (200) d'un dispositif de perfusion de médicament (102), pour savoir si un programme de perfusion (107, 217) est stocké dans une mémoire (201) du dispositif de perfusion de médicament, le dispositif de perfusion de médicament pouvant être connecté à un patient et administrer un médicament au patient selon le programme de perfusion sous la commande du dispositif de commande après configuration du dispositif de perfusion de médicament selon des paramètres spécifiés par le programme de perfusion ;
si le programme de perfusion est stocké dans la mémoire, l'incitation, par le dispositif de commande avec une première série d'invites, d'un utilisateur à confirmer l'utilisation du programme de perfusion, à effectuer une confirmation d'un identifiant de médicament et à examiner la configuration du dispositif de perfusion de médicament selon les paramètres spécifiés par le programme de perfusion ;
si le programme de perfusion n'est pas stocké dans la mémoire et qu'une zone de soins cliniques (106) dans laquelle le dispositif de perfusion de médicament est situé ou doit être utilisé, a été identifiée par un identifiant de zone de soins cliniques, l'activation, par le dispositif de commande, d'une capacité pour le dispositif de perfusion de médicament à recevoir et stocker dans la mémoire du dispositif de perfusion de médicament un programme automatique (113) avec lequel le dispositif de perfusion de médicament peut se configurer sans intervention de l'utilisateur selon des paramètres spécifiés par le programme automatique en tant que programme de perfusion ;
dans lequel ladite activation ouvre une fenêtre de temps pour recevoir et stocker le programme automatique pour la zone de soins cliniques identifiée,
si le programme automatique n'est pas reçu après que la capacité du dispositif de perfusion de médicament à recevoir et stocker le programme automatique est activée, l'attente, par le dispositif de commande, de la réception du programme automatique ou d'une sélection par l'utilisateur pour entrer manuellement dans le programme de perfusion ; et
lorsque le dispositif de commande commence à attendre la réception du programme automatique ou une sélection par l'utilisateur pour entrer manuellement dans le programme de perfusion, l'affichage, par le dispositif de commande, d'un écran de menu indiquant que le dispositif de perfusion de médicament attend le programme automatique ; et dans lequel si le programme automatique est reçu i) alors que la capacité du dispositif de perfusion de médicament à recevoir et stocker le programme automatique est activée et ii) avant que le dispositif de commande ne commence à attendre la réception du programme automatique ou une sélection par l'utilisateur pour entrer manuellement dans le programme de perfusion, alors l'écran de menu indiquant que le dispositif de perfusion de médicament attend le programme automatique, n'est pas affiché,
dans lequel la capacité du dispositif de perfusion de médicament à recevoir et stocker le programme automatique n'est pas activée par le dispositif de commande à moins qu'un programme de perfusion précédemment stocké en mémoire soit effacé ou terminé.

2. Procédé selon la revendication 1, comprenant en outre :
la réalisation, par le dispositif de commande, d'un autotest de mise sous tension du dispositif de perfusion de médicament ;
la réception du récipient du médicament dans un espace de réception verrouillable (209) à l'intérieur d'un boîtier (213) du dispositif de perfusion de médicament, le dispositif de commande pouvant recevoir un signal de réinitialisation indiquant que le récipient du médicament a été retiré de l'espace de réception verrouillable ;
si le signal de réinitialisation est reçu par le dispositif de commande après l'exécution de l'autotest de mise sous tension, l'arrêt, par le dispositif de commande, du procédé pendant n'importe quelle étape dans celui-ci et la réinitialisation, par le dispositif de commande, du procédé à un moment après l'exécution de l'autotest de mise sous tension ; et si le signal de réinitialisation n'est pas reçu par le dispositif de commande, l'exécution du procédé sans réinitialiser le procédé.

3. Procédé selon la revendication 1, dans lequel :
l'incitation, par le dispositif de commande avec la première série d'invites, comprend en outre :
l'incitation de l'utilisateur à sélectionner l'une des options suivantes : la confirmation de l'utilisation du programme de perfusion et l'effacement du programme de perfusion ; et
à la suite de la confirmation de l'utilisation du programme de perfusion sélectionné, l'incitation de l'utilisateur à confirmer l'identifiant de médicament et à examiner la configuration du dispositif de perfusion de médicament selon les paramètres spécifiés par le programme de perfusion ; et
le procédé comprend en outre :
si effacer le programme de perfusion est sélectionné, l'activation, par le dispositif de commande, de la capacité du dispositif de perfusion de médicament à recevoir le programme automatique.

4. Procédé selon la revendication 1, dans lequel :
le programme de perfusion spécifie également un identifiant de médicament de programme ; et le procédé comprend en outre :
la réception d'un flacon de médicament (215) dans un espace de réception verrouillable (209) à l'intérieur d'un boîtier (213) du dispositif de perfusion de médicament, le flacon de médicament présentant des indices spécifiant un identifiant de médicament du flacon ;
la détermination, par le dispositif de commande, de l'identifiant du médicament du flacon directement à partir des indices du flacon de médicament ;
si l'identifiant de médicament du flacon correspond à l'identifiant de médicament du programme, la réalisation, par le dispositif de commande avec la première série d'invites, de l'incitation de l'utilisateur à revoir la configuration du dispositif de perfusion de médicament selon les paramètres spécifiés par le programme de perfusion ; et si l'un des identifiants de médicament du flacon ne correspond pas à l'identifiant de médicament du programme, l'incitation, par le dispositif de commande avec une seconde série d'invites, de l'utilisateur à vérifier le flacon de médicament.

5. Procédé selon la revendication 4, comprenant en outre :
à la suite du retrait du flacon de médicament de l'espace de réception verrouillable, la génération, par un capteur de présence de flacon (207), d'un signal de réinitialisation du flacon ;
la réception, par le dispositif de commande, du signal de réinitialisation du flacon ; et
à la suite du signal de réinitialisation du flacon, la réinitialisation, par le dispositif de commande, d'un processus de configuration du dispositif de perfusion de médicament.

6. Dispositif de perfusion (102) comprenant :
une mémoire (201) et
un dispositif de commande (200) pour déterminer si un programme de perfusion (107, 217) est stocké dans la mémoire du dispositif de perfusion de médicament, le dispositif de perfusion de médicament pouvant être connecté à un patient et délivrer un médicament au patient selon le programme de perfusion sous la commande du dispositif de commande après configuration du dispositif de perfusion de médicament selon des paramètres spécifiés par le programme de perfusion ;
si le programme de perfusion est stocké dans la mémoire, l'incitation, par le dispositif de commande avec une première série d'invites, d'un utilisateur à confirmer l'utilisation du programme de perfusion, à effectuer une confirmation d'un identifiant de médicament et à examiner la configuration du dispositif de perfusion de médicament selon les paramètres spécifiés par le programme de perfusion ;
si le programme de perfusion n'est pas stocké dans la mémoire, et qu'une zone de soins cliniques (106) dans laquelle le dispositif de perfusion de médicament est situé ou doit être utilisé, a été identifiée par un identifiant de zone de soins cliniques, l'activation, par le dispositif de commande, d'une capacité pour le dispositif de perfusion de médicament de recevoir et stocker dans la mémoire du dispositif de perfusion de médicament un programme automatique (113) avec lequel le dispositif de perfusion de médicament peut se configurer sans intervention de l'utilisateur selon des paramètres spécifiés par le programme automatique en tant que programme de perfusion ;
dans lequel ladite activation ouvre une fenêtre de temps pour recevoir et stocker le programme automatique pour la zone de soins cliniques identifiée,
si le programme automatique n'est pas reçu après que la capacité du dispositif de perfusion de médicament à recevoir et stocker le programme automatique est activée, l'attente, par le dispositif de commande, de la réception du programme automatique ou d'une sélection par l'utilisateur pour entrer manuellement dans le programme de perfusion ; et
lorsque le dispositif de commande commence à attendre la réception du programme automatique ou une sélection par l'utilisateur pour entrer manuellement dans le programme de perfusion, l'affichage, par le dispositif de commande, d'un écran de menu indiquant que le dispositif de perfusion de médicament attend le programme automatique ; et dans lequel si le programme automatique est reçu i) alors que la capacité du dispositif de perfusion de médicament à recevoir et stocker le programme automatique est activée et ii) avant que le dispositif de commande ne commence à attendre la réception du programme automatique ou une sélection par l'utilisateur pour entrer manuellement dans le programme de perfusion, alors l'écran de menu indiquant que le dispositif de perfusion de médicament attend le programme automatique, n'est pas affiché, dans lequel la capacité du dispositif de perfusion de médicament à recevoir et stocker le programme automatique n'est pas activée par le dispositif de commande à moins qu'un programme de perfusion précédemment stocké en mémoire soit effacé ou terminé.

7. Dispositif de perfusion selon la revendication 6, comprenant en outre :
un moteur de communication (202) pour communiquer avec un serveur de gestion de médicaments (103).

8. Dispositif de perfusion selon la revendication 7,
dans lequel le moteur de communication est configuré pour transmettre et/ou recevoir le programme automatique, un programme de perfusion, des fichiers journaux et des messages.

9. Dispositif de perfusion selon l'une des revendications précédentes 6-8, comprenant en outre :
un clavier (203) permettant à l'utilisateur d'entrer ou de confirmer manuellement l'utilisation du programme de perfusion, ou d'effectuer une confirmation d'un identifiant de médicament, et
un dispositif d'affichage (204) pour afficher les invites et l'écran de menu.

10. Système d'administration de médicament (100) comprenant :
un serveur de gestion de médicaments (103) configuré pour générer un programme automatique (113) ou acheminer le programme automatique vers un dispositif de perfusion (102), et
le dispositif de perfusion, le dispositif de perfusion comprend,
une mémoire (201) et
un dispositif de commande (200) pour déterminer si un programme de perfusion (107, 217) est stocké dans la mémoire du dispositif de perfusion de médicament, le dispositif de perfusion de médicament pouvant être connecté à un patient et délivrer un médicament au patient selon le programme de perfusion sous la commande du dispositif de commande après configuration du dispositif de perfusion de médicament selon des paramètres spécifiés par le programme de perfusion ;
si le programme de perfusion est stocké dans la mémoire, inciter, par le dispositif de commande avec une première série d'invites, un utilisateur à confirmer l'utilisation du programme de perfusion, à effectuer une confirmation d'un identifiant de médicament et à examiner la configuration du dispositif de perfusion de médicament selon les paramètres spécifiés par le programme de perfusion ;
si le programme de perfusion n'est pas stocké dans la mémoire et qu'une zone de soins cliniques (106) dans laquelle le dispositif de perfusion de médicament est situé ou doit être utilisé, a été identifiée par un identifiant de zone de soins cliniques, activer, par le dispositif de commande, une capacité pour le dispositif de perfusion de médicament à recevoir et stocker dans la mémoire du dispositif de perfusion de médicament le programme automatique avec lequel le dispositif de perfusion de médicament peut se configurer sans intervention de l'utilisateur selon des paramètres spécifiés par le programme automatique en tant que programme de perfusion ;
dans lequel ladite activation ouvre une fenêtre de temps pour recevoir et stocker le programme automatique pour la zone de soins cliniques identifiée,
si le programme automatique n'est pas reçu après que la capacité du dispositif de perfusion de médicament à recevoir et à stocker le programme automatique est activée, attendre, par le dispositif de commande, la réception du programme automatique ou une sélection par l'utilisateur pour entrer manuellement dans le programme de perfusion ; et
lorsque le dispositif de commande commence à attendre la réception du programme automatique ou une sélection par l'utilisateur pour entrer manuellement dans le programme de perfusion, afficher, par le dispositif de commande, un écran de menu indiquant que le dispositif de perfusion de médicament attend le programme automatique ; et dans lequel si le programme automatique est reçu i) alors que la capacité du dispositif de perfusion de médicament à recevoir et stocker le programme automatique est activée, et ii) avant que le dispositif de commande ne commence à attendre la réception du programme automatique ou une sélection par l'utilisateur pour entrer manuellement dans le programme de perfusion, alors l'écran de menu indiquant que le dispositif de perfusion de médicament attend le programme automatique n'est pas affiché, dans lequel la capacité du dispositif de perfusion de médicament à recevoir et stocker le programme automatique n'est pas activée par le dispositif de commande à moins qu'une perfusion le programme précédemment stocké en mémoire soit effacé ou terminé.

11. Système d'administration de médicament selon la revendication 10, comprenant en outre :
au moins un autre ordinateur, poste de travail, serveur ou dispositif de communication en réseau (104).

12. Système d'administration de médicament selon l'une des revendications précédentes 10-11,
dans lequel le système d'administration de médicament comprend en outre une base de données (112).

13. Système d'administration de médicament selon la revendication 12,
dans lequel le serveur de gestion de médicaments est configuré pour générer le programme de perfusion sur la base d'une prescription.

14. Système d'administration de médicament selon la revendication 12,
dans lequel la base de données comprend au moins l'un de données de médicament décrivant les divers médicaments que le dispositif de perfusion de médicament peut délivrer à un patient, de paramètres admissibles pour programmer le dispositif de perfusion de médicament dans diverses conditions ou de paramètres admissibles dans diverses zones de soins cliniques.
